# EUROPEAN PATENT SPECIFICATION

(11) **EP 0 751 484 B1**
(45) Date of publication and mention of the grant of the patent: **02.01.2004**
(21) Application number: 96304280.9
(22) Date of filing: 07.06.1996
(51) Int. Cl.: G06T 11/00

(54) **Image reconstruction from helical partial cone-beam data**
Bildrekonstruierung von spiralabgetasteten Teilkegelstrahldaten
Reconstitution d'images des données de faisceaux coniques partiels à balayage spiral

(30) Priority: 30.06.1995 US 497296
(43) Date of publication of application: 02.01.1997
(73) Proprietor: Koninklijke Philips Electronics N.V., 5621 BA Eindhoven (NL)
(72) Inventor: Tuy, Heang K., Chesterland, Ohio 44026 (US)
(74) Representative: van der Veer, Johannis Leendert

(56) References cited:
- US-A- 5 337 231
- US-A- 5 377 250
- US-A- 5 404 293
- HIROYUKI KUDO ET AL: "THREE-DIMENSIONAL HELICAL-SCAN COMPUTED TOMOGRAPHY USING CONE-BEAM PROJECTIONS" SYSTEMS & COMPUTERS IN JAPAN, vol. 23, no. 12, 1 January 1992, pages 75-82, XP000380347
- CHO Z H ET AL: "WEIGHTED BACKPROJECTION APPROACH TO CONE BEAM 3D PROJECTION RECONSTRUCTION FOR TRUNCATED SPHERICAL DETECTION GEOMETRY" IEEE TRANSACTIONS ON MEDICAL IMAGING, vol. 13, no. 1, 1 March 1994, pages 110-121, XP000440154

## Description

The present invention relates to a diagnostic imaging method and apparatus. It finds particular application in conjunction with reconstructing x-ray transmission data from CT scanners which move a partial cone-beam radiation source along a helical trajectory and will be described with particular reference thereto. It is to be appreciated, however, that the present application will also find application in reconstructing information from other conical or other three-dimensional x-ray sources, such as reconstructing conical transmission or emission data in nuclear cameras.

Conventionally, spiral CT scanners include an x-ray source which projects a thin slice or beam of radiation. The x-ray source is mounted for rotational movement about a subject who is moving along the axis of rotation. An arc or ring of radiation detectors receive radiation which has traversed the patient. Data from the x-ray detectors represents a single spiralling slice through the patient. The data from the detectors is reconstructed into a three-dimensional image representation.

For faster processing, a pair or more of radiation detectors can be disposed next to each other. This enables two or more slices of data to be collected' concurrently. However, like the single slice scanner, only intraslice data is used in the reconstruction process.

One of the difficulties with such prior art scanners is that they place major stress on the x-ray generator. When a solid geometric shape of x-rays, such as a cone, are generated, the x-rays pass through a volumetric region of the subject. In true cone beam reconstruction, truncation of the data is not permitted. These x-rays pass along known rays, both within traditional planes and at acute angles through several planes. The radiation passing through rays at an angle to the central plane were previously lost to collimation. By utilizing the radiation previously lost in collimation to generate useful diagnostic information, the load on the x-ray generator is reduced.

However, images reconstructed from data collected along divergent beams tend to have artifacts. One way of minimizing the divergent ray artifacts is to minimize the number of rings, i.e., limit the width of the cone beam. Of course, limiting the width of the cone-beam partially defeats the original intent.

Although the additional radiation supplied by the cone-beam is beneficial in imaging, it has the detrimental side effect of increasing patient dosage. On the other hand, the high dosage enables a volume to be constructed with fewer rotations of the cone-beam.

In the article by H. Kudo and T. Saito: "Three-dimensional Helical-Scan Computed Tomography using Cone-Beam Projections" (Systems & Computers in Japan, vol. 23, no. 12, 1 January 1992, pages 75-82) an approximate algorithm for reconstructing volume images from cone-beam helical scanning with truncated projections is discussed. The presented approximate reconstruction algorithm is an extension to Feldkamp's reconstruction method for a one-rotation scan using cone-beam projections to a helical scan and consists of the three steps of multiplication by a weight function to correct the affect of a dispersed X-ray, convolution, and inverse projection. Feldkamp's reconstruction method is known to the person skilled in the art and does not address the general problem of truncated projections.

The present invention provides a new and improved method and apparatus for reconstructing volumetric images from cone and other three-dimensional x-ray geometries.

In accordance with the present invention, a method of diagnostic imaging is provided as described by claim 1.

During the reconstructing, the views are reconstructed based on the defined plane and voxel locations such that the views contribute to multiple planes to generate the volumetric image representation. The views are backprojected during reconstruction. Each view is backprojected into a three-dimensional volumetric region of the volumetric image representation in such a manner that each backprojected view contributes to a plurality of parallel planes of the volumetric image representation.

In accordance with another aspect of the present invention, an apparatus for diagnostic imaging is provided as described by claim 9.

One advantage of the present invention is that it provides for faster reconstruction of images of volumetric regions.

Another advantage of the present invention is that it enables volumetric images to be reconstructed with less than a full rotation of an x-ray source.

Another advantage of the present invention resides in reduced image artifacts.

The invention will now be further described, by way of example, with reference to the accompanying drawings in which: -
FIGURE 1a is an illustration of an imaging system in accordance with the present invention;
FIGURE 1b is an illustration of a detector element used in the present invention;
FIGURE 2 illustrates a local coordinate plane (P₀) associated with vertex φ(t₀);
FIGURE 3 further illustrates the local coordinate plane (P₀) associated with vertex φ(t₀) as shown in FIGURE 2;
FIGURE 4 illustrates a partial cone-beam data at any instance t;
FIGURE 5 illustrates a function hₜ(u,v) as an integral of a function f along the line from φ(t) to a point (u,v) in a plane defined by C(t) of the U and V axes;
FIGURE 6 illustrates a block diagram of a reconstruction algorithm for determining Q₂;
FIGURE 7 illustrates a graph of s(t)=2(1-cost)-tsint;
FIGURE 8 illustrates the trace of the helix on a local coordinate plane in (u,v) space;
FIGURE 9 illustrates M as the trace of x on (P₀) and D as the intersection of (Q) with (P₀);
FIGURE 10 is illustrates the integral of the function Hₜ along a line defined by polar parameter (s,ψ);
FIGURE 11 illustrates a coordinate system ω,q,r,s;
FIGURE 12 further illustrates the reconstruction algorithm as shown in FIGURE 6;
FIGURE 13 illustrates the backprojection process; and,
FIGURE 14 is a detailed view illustrating the processing of three-dimensional data for projection into a three-dimensional region of the volume image memory.

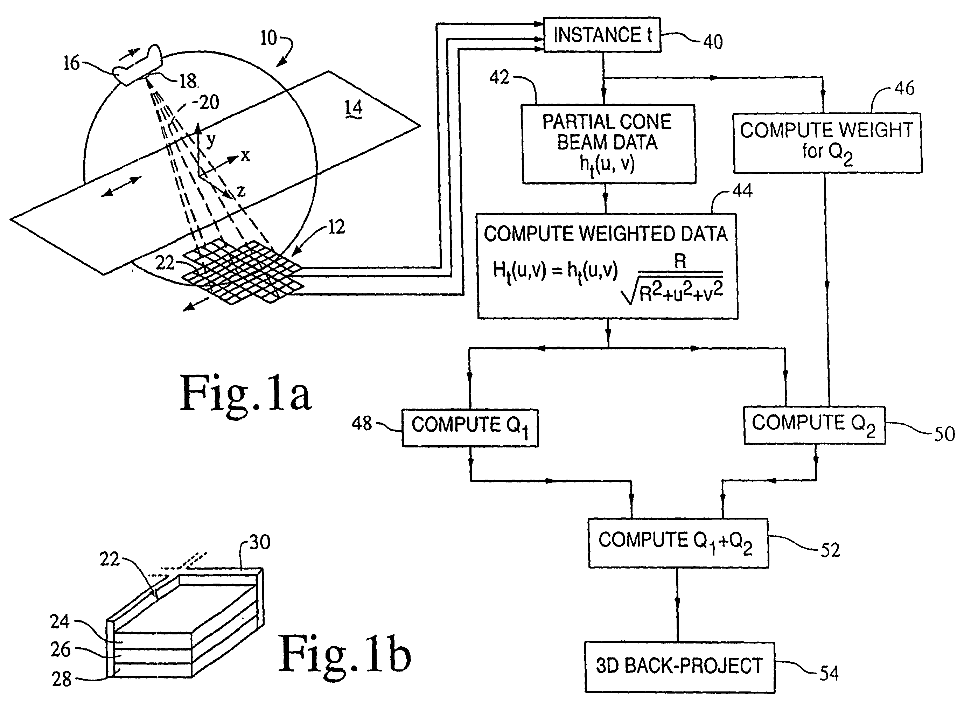

With reference to FIGURES 1a and 1b, a medical diagnostic imaging apparatus **10** includes an x-ray detector **12** which receives radiation arriving from a subject on a subject support **14**. In the preferred CT scanner embodiment, radiation emanates from an x-ray tube **16** having a source collimator **18** that defines a cone-beam **20** of radiation. The cone-beam, as defined herein, can have a circular cross-section, a square or rectangular cross-section, a hexagonal cross-section, or the like.

The radiation detector **12** of the preferred embodiment includes an array of detector elements mounted in a configuration which mimics the cross-section of the x-ray beam **20**. For example, a dozen square detector elements **22** may be mounted in a pattern which approximates a circle. When the beam is rectangular or square, the detector elements would be positioned in a corresponding square or rectangular pattern. As yet another alternate embodiment, a plurality of stationary rings of the detector elements can be mounted around the subject. Providing a plurality of stationary rings of the detector elements adds cost in the detectors, but removes the cost associated with rotating th e detector assembly.

Each of the detector assemblies **22** includes a scintillation crystal **24** disposed toward the radiation source, an array of photodetectors **26** disposed to view the scintillation crystal, and an array of integrated circuits **28** connected with the array of photodetectors **26**. Preferably, the scintillation crystal, photodetector, and integrated circuit assembly is mounted on a common substrate. The scintillation crystal and photodetectors are etched or cut to define a larger number of light-sensitive elements, e.g., a 16x16 array. Optionally, a detector collimator **30** is mounted to the scintillation crystals to limit received radiation to radiation travelling along rays from an origin of the cone-beam collimator. In the CT scanner embodiment, the origin of the collimator is selected to match the focal point of the x-ray tube.

Alternately, the medical diagnostic apparatus may be a nuclear camera. In the nuclear camera, the radiation source includes an emission radiation source in the form of a radiopharmaceutical that is injected into the subject. Because the cone-beam collimator limits received radiation to radiation travelling along a conical array of rays, the resultant data is again cone-beam data. Further, a transmission line source may be disposed opposite the patient from the radiation detector. In a nuclear camera, the transmission line source is commonly a radioisotope.

As is known in the art, an appropriate means or mechanical mechanism is provided for rotating the radiation source and the detector around the subject and subject support. In a CT camera, the radiation source commonly rotates continuously at a relatively high rate of speed. In a nuclear camera, the detector and transmission radiation source, if any, commonly rotate in incremental steps.

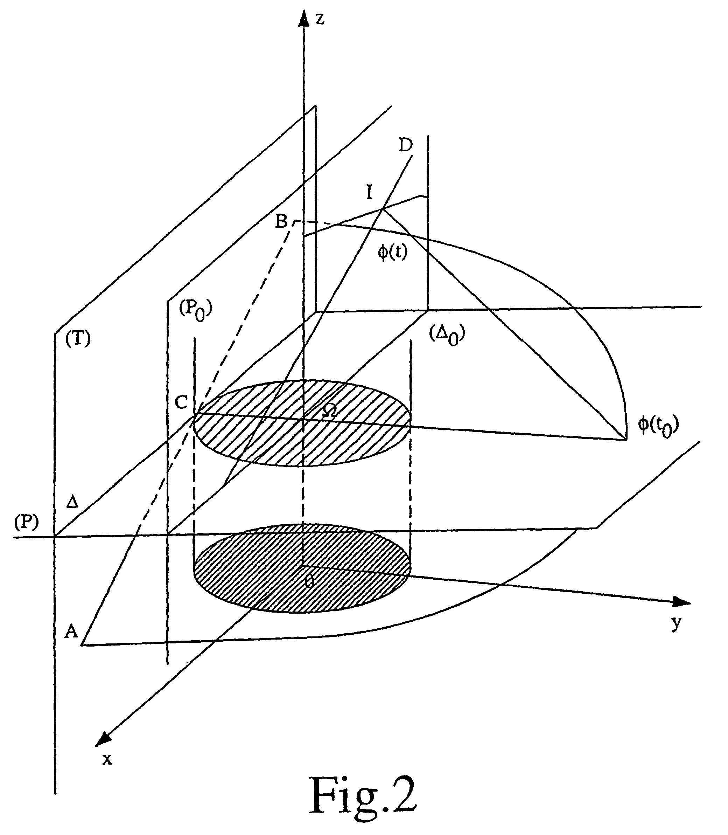

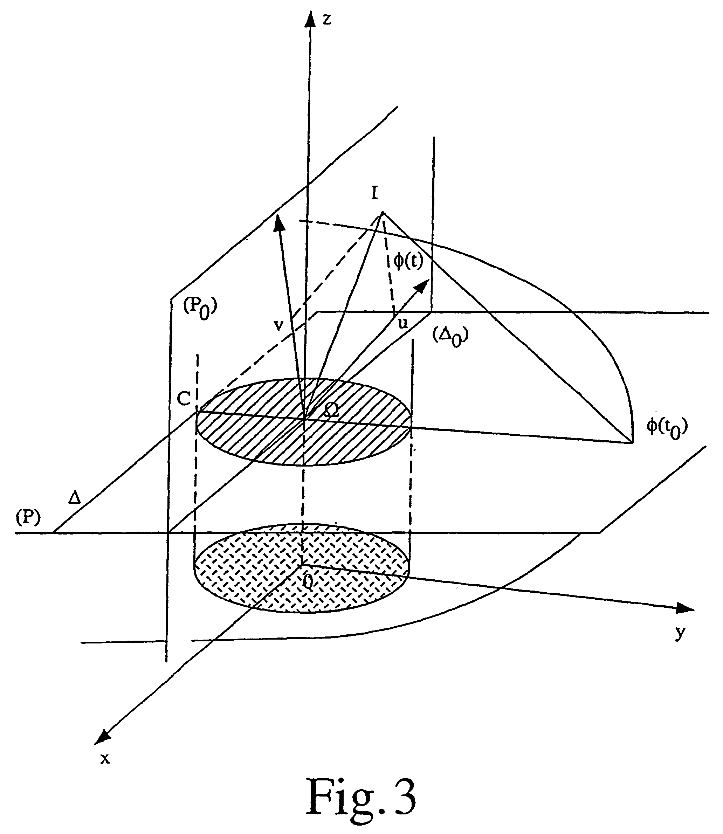

With continuing reference to FIGURES 1a-1b and further reference to FIGURES 2 and 3, the radiation detectors are all sampled concurrently to generate a current view which is stored in a current view memory or latch **40**. The memory or latch **40** also stores an indication of the origin of the cone, i.e., the location of the x-ray tube and/or the location of the detector assembly. In a CT scanner in which the cone is rotating rapidly, the detectors are sampled at very short time intervals. In a nuclear camera, the output of the detectors is commonly integrated over a dwell duration at each angular position. The data from the current view memory **40** is conveyed to a partial cone-beam data memory **42** which stores a source cone or fan of data hₜ(u,v). More specifically, the partial cone beam data hₜ represents the line integrals or projections onto the plane P₀ along the rays φ from the origin or x-ray source. A weighting processor **44** weights each partial cone data value hₜ(u,v) to generate a weighted or data view Hₜ(u,v). In a preferred embodiment, the weighting is cosine weighting of the form:${\text{H}}_{\text{z}} {\text{(u,v) = h}}_{\text{t}} \text{(u,v)} \frac{\text{R}}{\sqrt{{\text{R}}^{\text{2}} {\text{+u}}^{\text{2}} {\text{+v}}^{\text{2}}}}$ where the weighting term is the cosine of the ray angle and 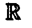 is the radius of the circle that defines the scan helix.

The object to be reconstructed is represented by a function f from 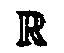 ³ to 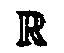 , where 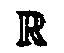 is the radius of a circle that defines the scan helix, i.e., the distance from the z-axis at the origin Ω to the origin of the cone. The trajectory of the x-ray source is described by a curve φ which is defined mathematically by a function φ from an interval Λ of 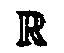 to 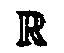 ³. In the case of helical scanning, such a function φ is defined by three coordinate equations φ(t)=(Λ cos ωt, Λ sin ωt, σt) for t a subset of Λ. For any direction represented by a unit vector α in 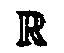 ³, the positive half-line originated at φ(t) can be represented parametrically by {φ(t) + rα | r ∈ [0,∞]} so that h(α,t) is defined by: 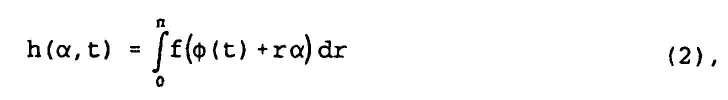 is the integral of the function f along the semi-line starting at φ(t) in the direction α. A partial cone-beam data at any instant t₀ may be represented by h(α,t), with α in the subset of 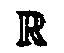 ³. φ(t) is the vertex of the partial cone at the particular instant t₀. The definition of the function h can be extended for all α in 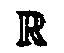 ³ with ∥ α ∥ ≠ 0. Such an extended version of h is denoted hereinafter by g. The inner product of two vectors x,y in 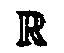 ³ is denoted by <x,y>. The Fourier transform of the function f is given by: 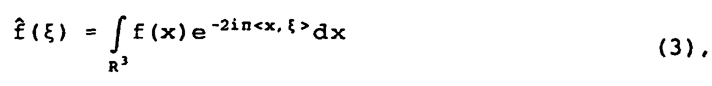 or in spherical coordinates: 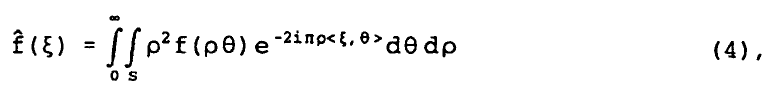 where S denotes the unit sphere R³.

The following equations give a relationship between the Fourier transform of a function f and that of a function g:$\text{G(ξ,t) = F(ξ,<φ(t),ξ>)}$ where, 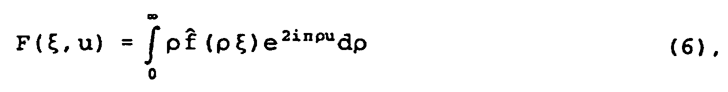 and 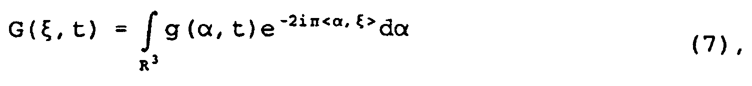 is the three-dimensional Fourier transform of g with respect to the first variable α. From the definition itself of the function F, it can be concluded that: 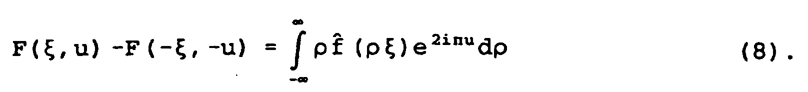 If θ is a unit vector in R³, the slice projection theorem which produces the relationship between the Fourier transform of f and the one-dimensional Fourier transform of its planar integrals R_{θ}f, it can be concluded that:$\text{F(θ,u)-F(-θ, -u) =} \frac{\text{1}}{\text{2iπ}} {\text{(R}}_{\text{θ}} \text{f)'(u)}$ Consequently, the expression F(θ,u) - F(-θ,-u) is 0 if |u|>A for A, because the integrals along planes perpendicular to θ are 0 if the plane does not intersect the support of the function f. From this, it can be concluded that: 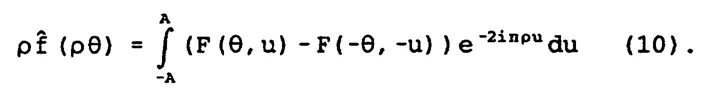 In condition 1, it is assumed that for any point x in the support of the function f, where f(x) is to be reconstructed from the cone-beam data, there exists a sub-interval Λₓ of Λ such that any plane going through the support of f intersects the subcurve φₓ of φ associated with the sub-interval Λₓ. From this condition and by making the change of variables defined by u = <φ(t),θ>, Equation (10) can be expressed as: 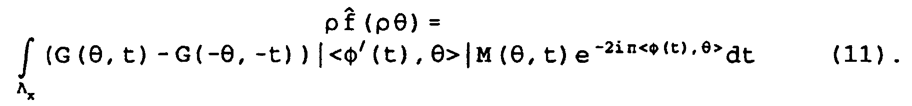 where the redundancy weight function M(θ,t) satisfies the following multiplicity condition which pertains to the number of times that the plane going through φ(t) and perpendicular to θ cuts the curve φₓ: 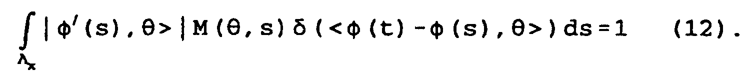 If the plane perpendicular to a direction θ and going through φ(t) intersects the sub-curve φₓ at φ(s₀), ..., φ(s_{tθ}), then the condition of Equation (12) can simply be expressed as:${\text{M(θ, s}}_{\text{0}} {\text{) + M(θ, s}}_{\text{1}} {\text{) + ... + M(θ,s}}_{\text{tθ}} \text{) = 1}$ The term f(x) can be computed from its Fourier transform using the spherical coordinate system as follows: 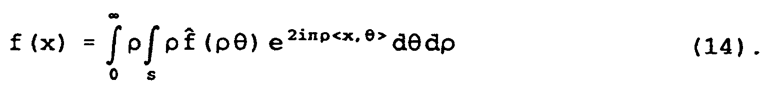 Substituting Equation (11) into Equation (14), one obtains: 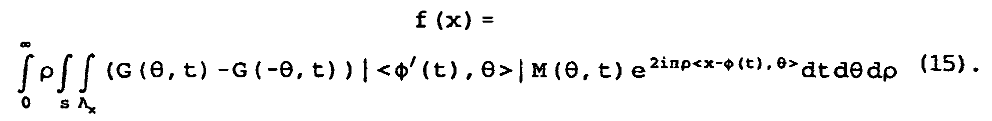 After interchanging the order of integration, one obtains: 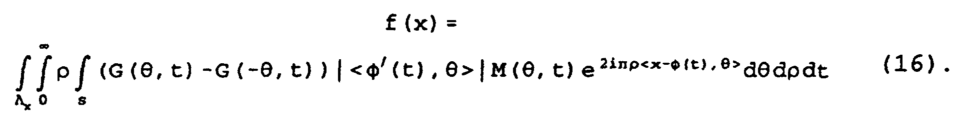 Observing that M(-θ,t) = M(θ,t), after the integration with respect to ρ, one has: 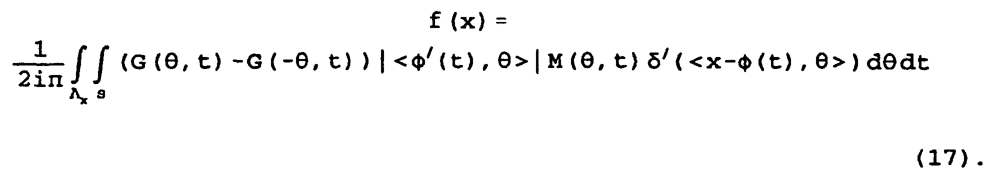 Consequently, the computation of f(x) requires:${\text{Λ}}_{\text{x}} \text{= {t∈Λ|<x-φ(t),θ> = 0}}$ that is, the set of t is such that φ(t) is the intersection of the plane going through f and perpendicular to the direction θ. The expression f(x) can also be written: 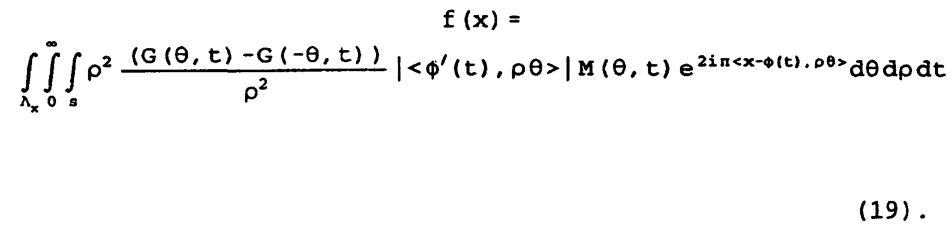 Observe that g and G are both homogeneous functions with respect to their first variable, and that G(ρθ,t) = G(θ,t)/ρ², Equation (19) can be written as:

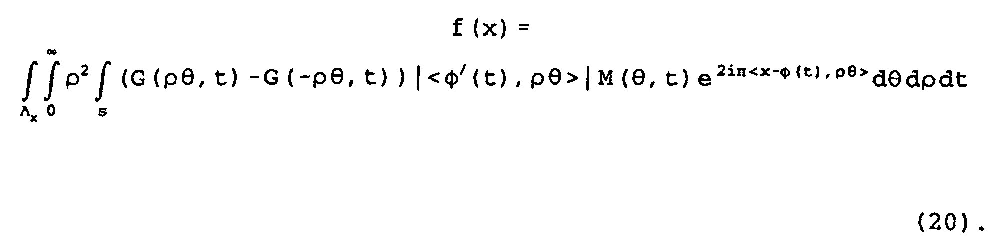

The inner double integrals of the right hand side of Equation (20) is only the three-dimensional inverse Fourier transform of the product of two functions, namely: G(ξ,t) - G(-ξ,t), and |<φ'(t), ξ>|M(ξ/∥ξ∥, t). Consequently, the integrals are the three-dimensional convolution of their inverse Fourier transform. More precisely: 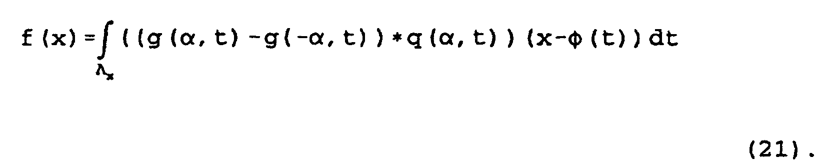 with 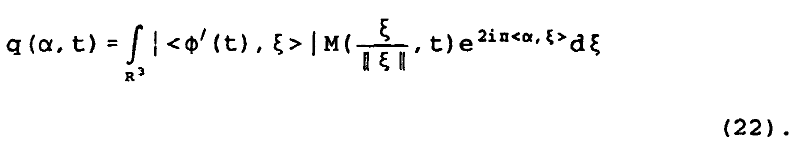 The above convolution backprojection is complex due to the redundancy of the weighting function M and is not appropriate for partial cone-beam data.

For the weighting function M constant, and with such constant set equal to 1 for simplicity, the kernel of the convolution is given by: 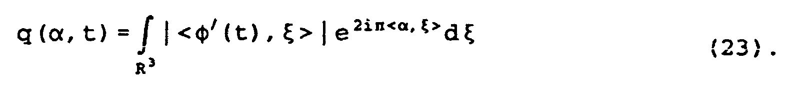 The expression α and ξ in the coordinate system (T,U,V) with the T axis going through φ(t), and the U axis parallel to the vector φ'(t): 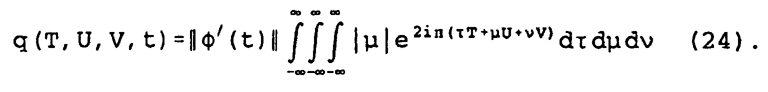 Hence,$\text{q(T,U,V,t) =-} \frac{\left||\text{φ'(t)}\right||}{{\text{2π}}^{\text{2}} {\text{U}}^{\text{2}}} {\text{δ}}_{\text{T}} {\text{δ}}_{\text{V}}$ i.e., q is the product of the classical ramp filter for the two-dimensional reconstruction with the delta function with respect to the variable (T,V) weighted with ∥φ'(t)∥. Consequently, the expression Q(x,t) = (g(α,t)*q(α,t))(x-φ(t)) becomes: 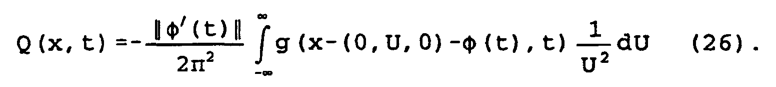 Note that the function g is homogeneous with respect to its first variable. The convolution expression is a one-dimensional convolution of weighted line integrals or projection data along the direction of the tangent φ'(t) of the trajectory φ. The kernel of the convolution is the classical ramp kernel which is used in two-dimensional slice reconstruction. Furthermore, the convolution of g(-α,t) is null since the object is of compact support. Thus, if M is constant, i.e., every plane goes through the point x at which f(x) is to be reconstructed cuts a sub-curve φₓ at the same number of points, then the convolution is a one-dimensional convolution in the direction of φ'(t). Consequently, data can be collected in a partial cone-beam, as long as the data are not truncated in the direction of φ'(t), for any instance t in Λₓ.

Although the exact reconstruction formula requires a non-constant weighting function M(θ,t) which satisfies the redundancy condition stated above, based on the simplicity of the convolution expression, the weighting function M can be written as the sum of a constant C and a non-constant function N, i.e.:$\text{M(θ, t) = C + N(θ,t)}$ With this decomposition, the convolved data is the sum of two terms. The first term corresponds to C; and the second term corresponds to the weighting function N. Because the first term requires a one-dimensional convolution in the direction φ'(t), it can be assumed that the partial cone-beam data is collected within a rectangle with one side parallel to φ'(t).

In condition 2, it is assumed that at any instant t, the data of the partial cone beam is not truncated in the direction φ'(t) and there exists a positive integer K. The integer K has the property that if at time t, the data is truncated along a direction Δ, then the plane defined by the line Δ and the vertex φ(t) either (i) intersects φₓ exactly K times or (ii) intersects φₓ at another vertex φ(t') for which the data along the line Δ' orthogonal to the plane is not truncated.

Furthermore, it is assumed that along that side, the data is not truncated. The redundancy condition on the function M requires N to satisfy the following condition: 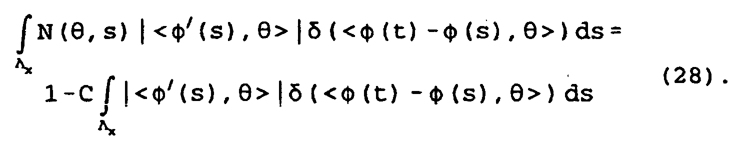 Observe that if W(θ,t)≥0 and satisfies the condition: 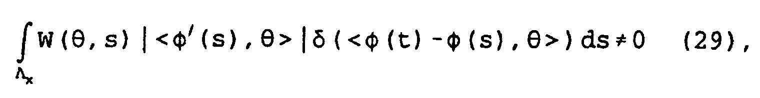 then the function N can be defined by: 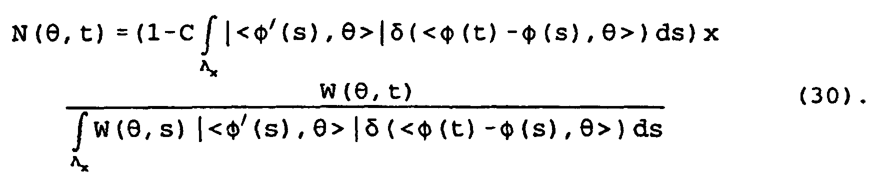 W is chosen such that the computation of the convolved data does not use the truncated data. Using the truncated data would allow N(θ,t) = 0.
Thus, to simplify the reconstruction, the reconstruction formula is chosen to be: 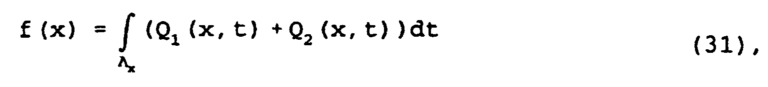 where Q₁ is the convolved data corresponding to C which is the one-dimensional convolution of the weighted data with the classical ramp function in the direction φ'(t) and the second term Q₂ is given by: 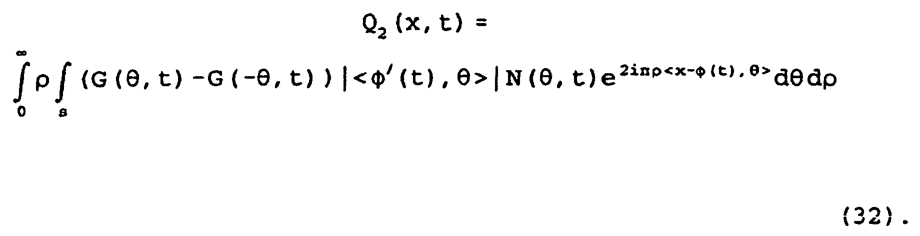 Thus, it follows that: 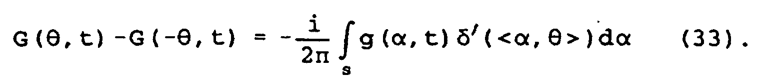 The right hand side of Equation (33) is only the product of -i/2π, with the integral of the directional derivative of propagation data along a line in the detector plane and orthogonal to the direction θ. Consequently, the computation of Q₂ does not require the data along that line with the truncated data. To insure that such choice is always possible, W is selected accordingly. For this reason, appropriate conditions are set on the curve φ traversed by the x-ray source or origin of the cone-beam relative to the axis z. A spiral or a partial spiral extending from A to B satisfies the conditions.

The weighting processor **44** performs the constant weighting component weighting of the data while a second factor processor **46** calculates the non-constant weighting factor. The weighted data from the processor **44** is conveyed to a first or Q₁ processor **48** which calculates the Q₁ component and to a second or Q₂ processor **50** which calculates the Q₂ component with the non-constant weighting function from the weighting function processor **46.** A processor **52** computes the sum of Q₁ and Q₂ and the sum is backprojected by a three-dimensional backprojector **54,** generally as described by Equations (19)-(21) and as described in greater detail below.

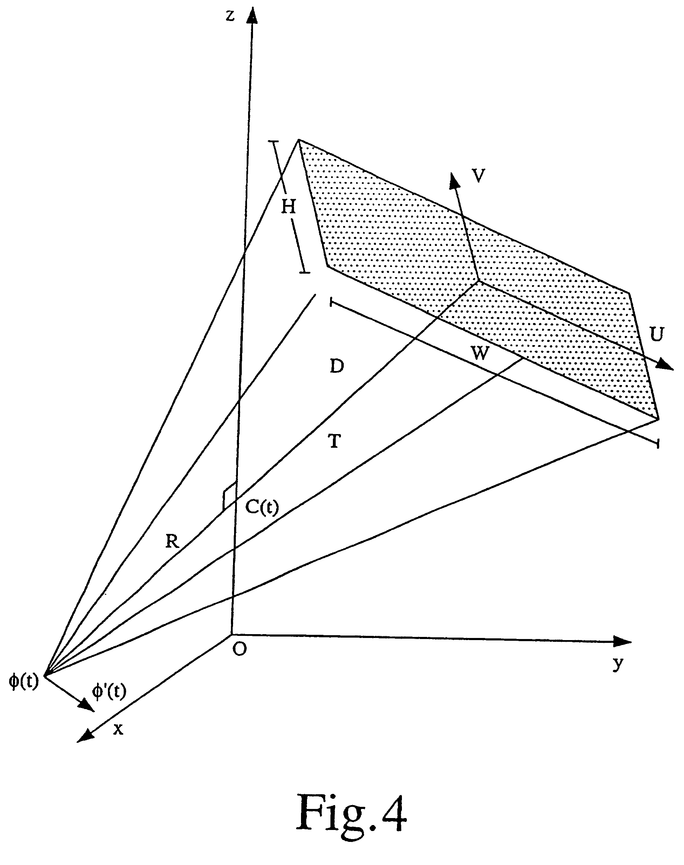

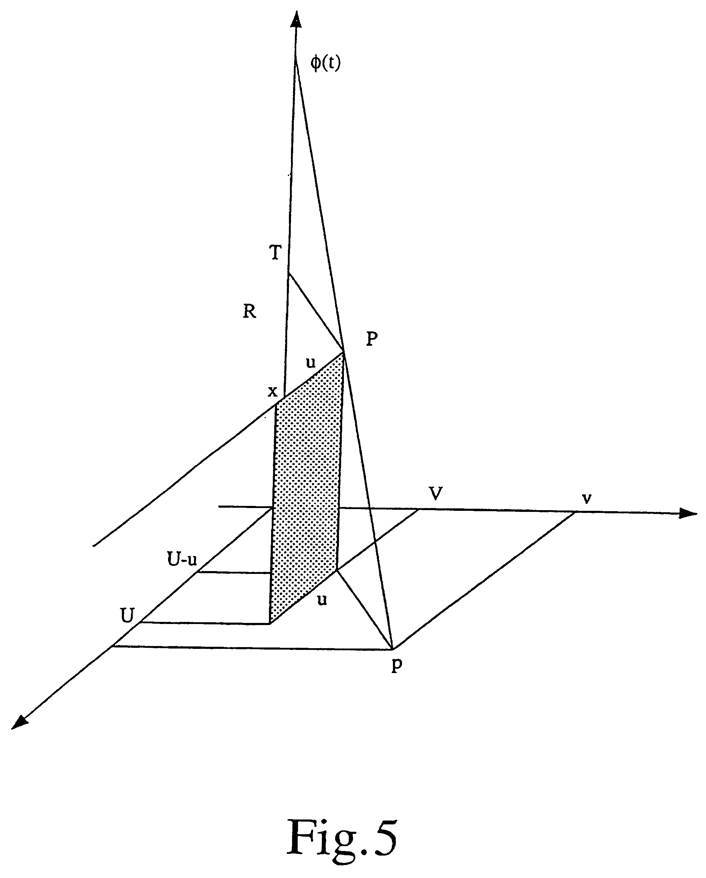

With reference to FIGURES 4, 5, and 6, we start first in the (T,U,V) coordinate system. In this coordinate system Q₁ is evaluated as: 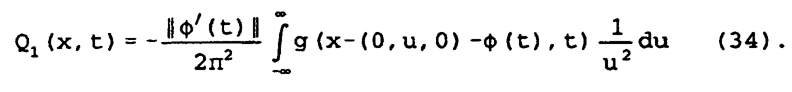 To simplify the coordinate transformation, the center of the coordinate system (T,U,V) is chosen to be the orthogonal projection C(t) of φ(t) onto the z-axis. This coordinate system is simply the translation of the coordinate system of FIGURE 5, with the translation vector D_{τ}, where τ is the unit vector along the T-axis. In this new coordinate system, the coordinates (T,U,V) of the reconstruction point x can be obtained from its original coordinates (X,Y,Z) by multiplying the column matrix (X,Y, Z-σt)^{t} with the 3x3 rotation matrix A(t) which consists of the coordinates of the unit vectors τ,µ,ν along the T,U, and V-axes respectively. For φ(t)=(Rcosωt, Rsinωt, σt) :$\text{τ = [cosωt sinωt 0]}$${\text{µ = [-Rωsinωt Rωcosωt σ]/(R}}^{\text{2}} {\text{ω}}^{\text{2}} {\text{+σ}}^{\text{2}} {\text{)}}^{\text{1/2}} \text{and}$${\text{ν = [σsinωt -σcosωt Rω]/(R}}^{\text{2}} {\text{ω}}^{\text{2}} {\text{+σ}}^{\text{2}} {\text{)}}^{\text{1/2}}$

It remains then to evaluate the integral expression of Q₁ using a sample from the set of line integrals hₜ which are estimated from the partial cone-beam projection data. Conforming to the choice of the new local coordinate system, hₜ(u,v) is defined as the integral of the function f along the line from φ(t) to the point (u,v) in the plane defined by C(t), the U and V-axes of the local coordinate system. For P as the point x-(0,u,0) as shown in FIGURE 3, by the homogeneity property of g, one can express g from the corresponding line integrals as follows:$\text{g(x-(0,u,0)-φ(t),t) =} \frac{\text{1}}{\left||\text{P-φ(t)}\right||} \text{g(} \frac{\text{P-φ(t)}}{\left||\text{P-φ(t)}\right||} \text{, t)}$ For p as the intersection of the line from φ(t) to P with the (C(t),U,V) plane, the coordinates of the point p in the coordinate system are (0,w(U-u),wV) with w=R/(R-T). Because p=φ(t)+w(P-φ(t))=(R,0,0)+ w(T-R,U-u,V), and because w is the constant which makes the first coordinate of p null, then:$\text{p-φ(t) = w(P-φ(t))}$ Hence,$\frac{\text{1}}{\left||\text{p-φ(t)}\right||} \text{=} \frac{\text{w}}{\left||\text{p-φ(t)}\right||}$ or:$\frac{\text{1}}{\left||\text{p-φ(t)}\right||} \text{=} \frac{\text{w}}{\sqrt{{\text{R}}^{\text{2}} {\text{+ w}}^{\text{2}} {\text{(U-u)}}^{\text{2}} {\text{+ w}}^{\text{2}} {\text{V}}^{\text{2}}}}$

Substituting into Equation (36):$\text{g(x-(0,u,0)-φ(t),t) =} \frac{\text{w}}{\sqrt{{\text{R}}^{\text{2}} {\text{+ w}}^{\text{2}} {\text{(U-u)}}^{\text{2}} {\text{+ w}}^{\text{2}} {\text{V}}^{\text{2}}}} {\text{h}}_{\text{t}} \text{(w(U-u),wV)}$

Accordingly, the expression of Q₁ becomes:

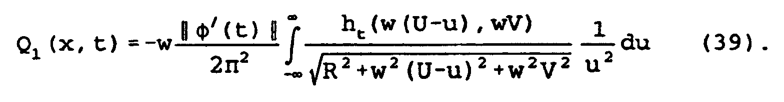

When v is defined as equal to wV, and after a change of the variable of integration is made, Q₁ becomes: 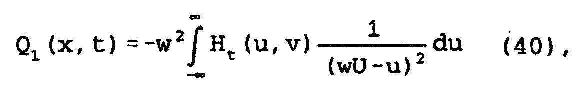 where Hₜ(u,v) is defined by Equation (1), where R=∥φ'(t)∥/2π². In other words, Q₁(x,t) is obtained by convolving Hₜ(u,v) with the classical ramp kernel with respect to the first variable u and evaluating it at wU, where U is the second coordinate of x in the local coordinate system. Hₜ(u,v) is the projection data hₜ(u,v) weighted with the constant divided by the distance from φ(t) to the point (u,v) on the plane (C(t),U,V).

Thus, the term Q₁ is essentially a one-dimensional convolution of the weighted data with the classical ramp 2D reconstruction filter. By contrast, the computation of Q₂ involves the computation of a weighting function N based on a knowledge of the number of intersection points of a plane with a portion of the helix φ, the trajectory of the vertex of the cones. Accordingly, before calculating Q₂, one first establishes the number of intersection points.

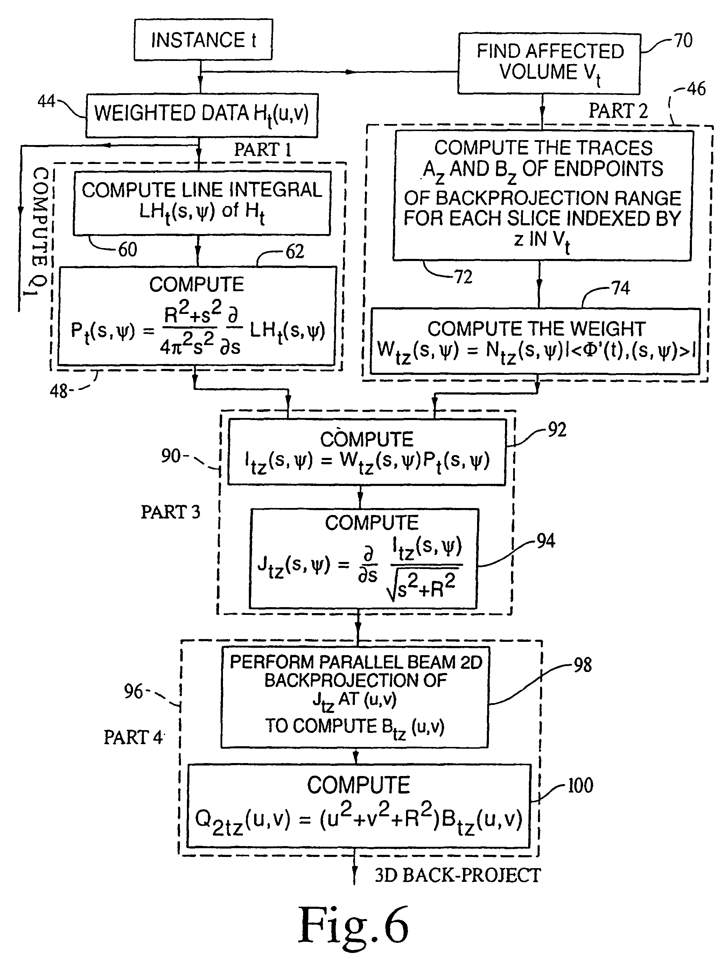

With reference again to FIGURES 1 and 2, and further reference to FIGURE 6, the equation for the local coordinate planes (cosωt₀, sinωt₀, 0) is:${\text{xcosωt}}_{\text{0}} {\text{+ ysinωt}}_{\text{0}} \text{=0}$

A plane (Q) containing φ(t₀) and a point x of the support of the object intersects φ at another point φ(t) if and only if the intersection I of the line joining the two vertices φ(t) and φ(t₀) with the coordinate plane (P₀) associated with φ(t₀) belongs to the intersection line D of the plane (Q) with the plane (P₀). Note that I belongs to both planes (Q) and (P₀). Conversely, when the intersection line D of (Q) with (P₀) contains the point I which is the intersection of φ(t₀),φ(t) with the plane (P₀), then φ(t) belongs to (Q), i.e., (Q) intersects φ at φ(t). To count the number of intersection points of the plane (Q) besides φ(t₀), the number of intersections points of the line D with the trace of the helix on (P₀) is counted.

The trace of φ on the local coordinate plane (P₀) associated with φ(t₀) is the set of intersection points I of the line φ(t₀)φ(t) with the plane (P₀) as t varies in an interval containing t₀. Similarly, the trace of a point x onto the local coordinate plane (P₀) is defined by the point of the intersection M at the line φ(t₀),x with the plane (P₀).

The trace of φ contains two branches of curves which intersect the u-axis at infinity, because the line φ(t₀)φ(t) becomes tangent to φ as t tends to t₀. The coordinates of the intersection point I can be written as a point of the line φ(t₀)φ(t) as:${\text{I = (Rcosωt}}_{\text{0}} {\text{+rR(cosωt-cosωt}}_{\text{0}} \text{),} {\text{Rsinωt}}_{\text{0}} {\text{+rR(sinωt-sinωt}}_{\text{0}} \text{),} {\text{σt}}_{\text{0}} {\text{+rσ(t-t}}_{\text{0}} \text{))}$ where r is a real number. Because the point I belongs to the local coordinate plane, the coordinates of I must satisfy the above equation of the local coordinate plane. Accordingly, r=(1-cosω(t-t₀))⁻¹. The Equation (42) coordinates of I are with respect to the original coordinate system Oxyz.

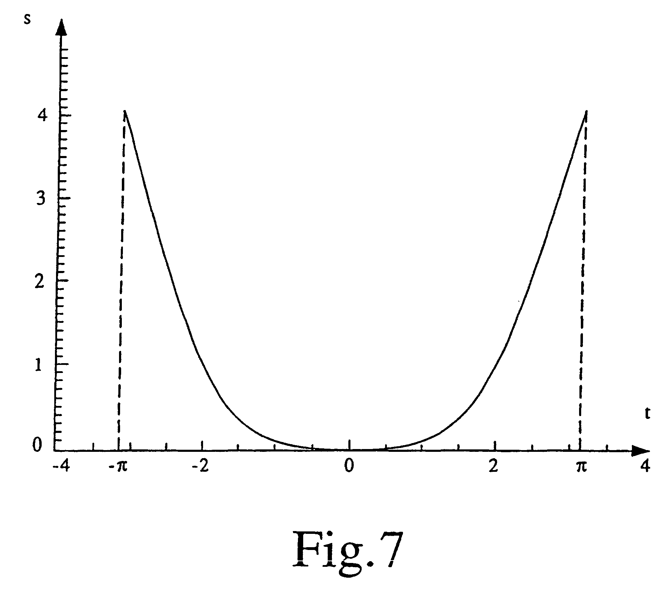

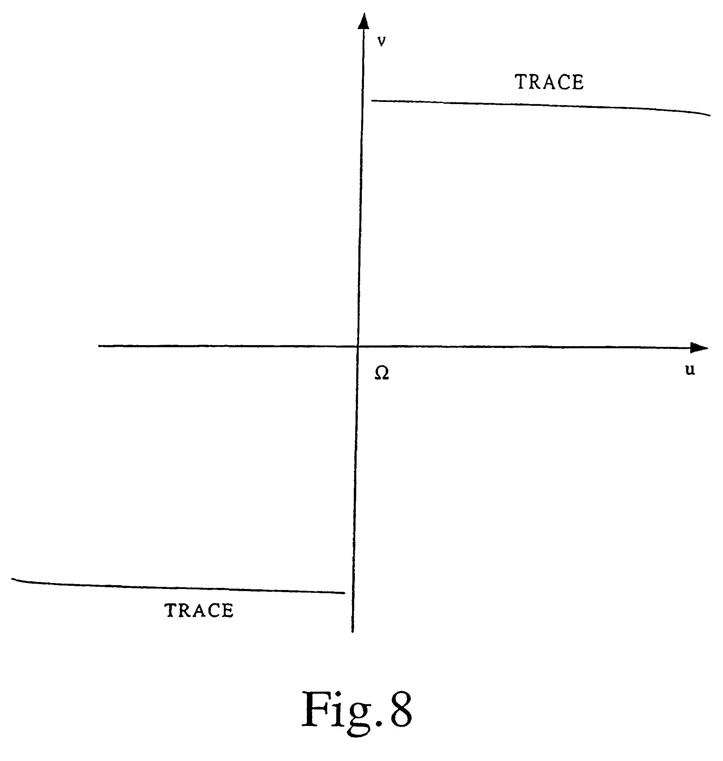

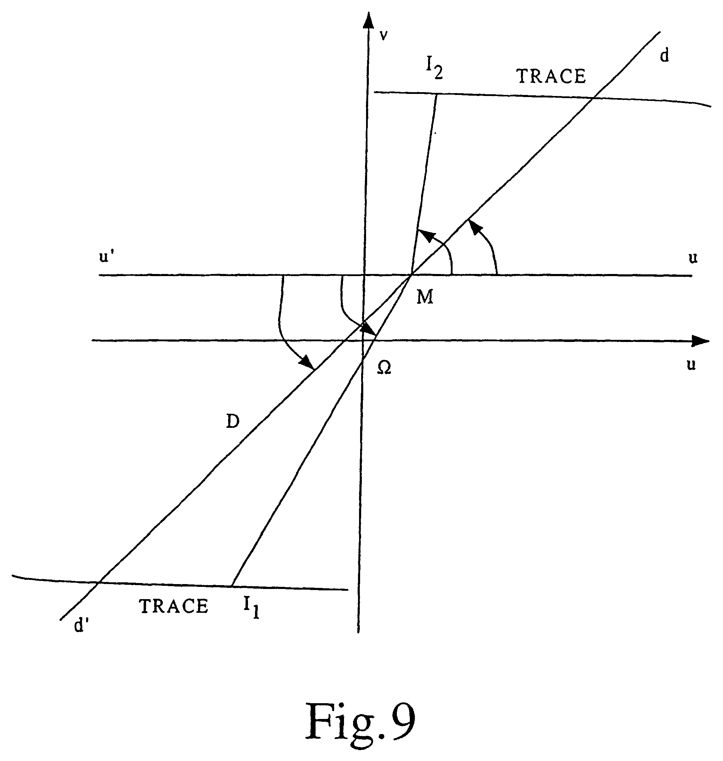

With reference to FIGURE 3, to obtain the coordinates (u,v) of I with respect to the local coordinate system, it suffices to compute the inner product of the vector ΩI with the unit vectors:${\text{µ= [-Rωsinωt}}_{\text{0}} {\text{Rωcosωt}}_{\text{0}} {\text{σ]/(R}}^{\text{2}} {\text{ω}}^{\text{2}} {\text{+σ}}^{\text{2}} {\text{)}}^{\text{1/2}} \text{and}$${\text{ν = [σsinωt}}_{\text{0}} {\text{-σcosωt}}_{\text{0}} {\text{Rω]/(R}}^{\text{2}} {\text{ω}}^{\text{2}} {\text{+σ}}^{\text{2}} {\text{)}}^{\text{1/2}}$ More specifically, u=<ΩI,µ> and v=<ΩI,ν>. Because:${\text{ΩI = (Rcosωt}}_{\text{0}} {\text{+ rR (cosωt - cosωt}}_{\text{0}} \text{),} {\text{Rsinωt}}_{\text{0}} {\text{+ rR (sinωt - sinωt}}_{\text{0}} \text{),} {\text{rσ(t - t}}_{\text{0}} \text{))}$ it follows that:$\text{u = r} \frac{{\text{R}}^{\text{2}} {\text{ωsinω (t - t}}_{\text{0}} {\text{) + σ}}^{\text{2}} {\text{(t - t}}_{\text{0}} \text{)}}{\sqrt{{\text{R}}^{\text{2}} {\text{ω}}^{\text{2}} {\text{+ σ}}^{\text{2}}}}$ and,$\text{v = Rσr} \frac{{\text{sinω (t}}_{\text{0}} {\text{- t) + ω (t - t}}_{\text{0}} \text{)}}{\sqrt{{\text{R}}^{\text{2}} {\text{ω}}^{\text{2}} {\text{+ σ}}^{\text{2}}}}$ as t→t₀, u→±∞, and v→0, for t=t₀±π/ω, Equations (45a) and (45b) become:$\text{u = ±} \frac{{\text{σ}}^{\text{2}} \text{π}}{\text{2ω} \sqrt{{\text{R}}^{\text{2}} {\text{ω}}^{\text{2}} {\text{+ σ}}^{\text{2}}}}$$\text{v = ±} \frac{\text{Rσπ}}{\text{2} \sqrt{{\text{R}}^{\text{2}} {\text{ω}}^{\text{2}} {\text{+ σ}}^{\text{2}}}}$ The derivatives of u and v with respect to t are:$\frac{\text{du}}{\text{dt}} \text{=} \frac{{\text{(σ}}^{\text{2}} {\text{- R}}^{\text{2}} {\text{ω}}^{\text{2}} {\text{) (1 - cosω (t- t}}_{\text{0}} {\text{)) - σ}}^{\text{2}} {\text{ω (t - t}}_{\text{0}} {\text{) sinω (t - t}}_{\text{0}} \text{)}}{\sqrt{{\text{R}}^{\text{2}} {\text{ω}}^{\text{2}} {\text{+ σ}}^{\text{2}}} {\text{(1 - cosω (t - t}}_{\text{0}} {\text{))}}^{\text{2}}}$$\frac{\text{dv}}{\text{dt}} \text{=} \frac{\text{Rσω}}{\sqrt{{\text{R}}^{\text{2}} {\text{ω}}^{\text{2}} {\text{+ σ}}^{\text{2}}}} \frac{{\text{(2 (1 - cosω (t- t}}_{\text{0}} {\text{)) - ω (t - t}}_{\text{0}} {\text{) sinω (t - t}}_{\text{0}} \text{))}}{{\text{(1 - cosω (t - t}}_{\text{0}} {\text{))}}^{\text{2}}}$ For ω and σ>0, the derivative of u with respect to t is negative, and that the derivative of v is positive. Note FIGURE 7. This analysis of u and v shows that the trace of the helix on a local coordinate plane has a graph in the (u,v) space as illustrated in FIGURE 8. This analysis suggests the following practical criteria to count the number of intersection points of a plane going through a point of reconstruction with part of a helix. With reference to FIGURE 9, [t₁, t₂] is an interval containing t₀ and I₁, I₂ is the trace of φ(t₁) and φ(t₂) on the local coordinate planes (P₀) associated to t₀, respectively. (Q) is a plane containing φ(t₀) and a point x on the support of the object. M is the trace of x on (P₀) and D is the intersection of (Q) with (P₀).

Looking to the first criteria, (Q) intersects φ at φ(t) with t in [t₁, t₂] if and only if D intersects the portion of the branch of the trace of the helix from -∞ to I₁. Practically, this is equivalent to the angle between the half lines Mu' and Md' being less than the angle between Mu' and MI₁.

Looking to the second criteria, (Q) intersects φ at φ(t) with t in [t₀, t₂] if and only if D intersects the portion of the branch of the trace of the helix from I₂ to ∞. Practically, this is equivalent to the angle between the half lines Mu and Md being less than the angle between Mu and MI₂.

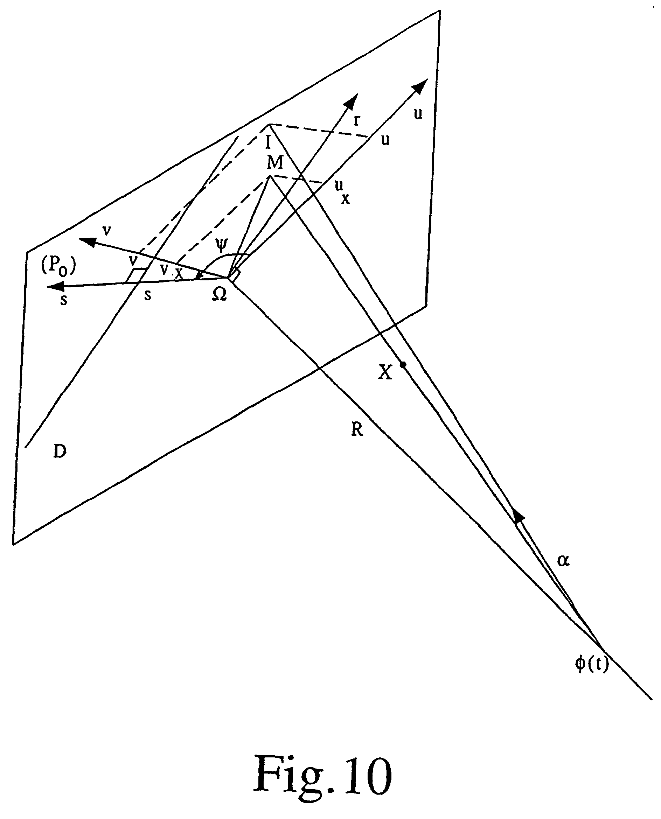

Q₂ as defined in Equations (32) and (33) can be further quantified by splitting the unit sphere into the union of two disjoint sphere halves, namely S/2 and - S/2, the upper and lower half unit spheres. Equation (32) then reduces to: 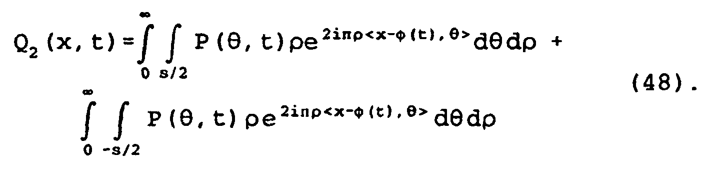 where:$\text{P(θ, t) = (G(θ,t)-G(-θ,t))|<φ'(t),θ>|N(θ,t)}$ Substituting -θ for θ in the second integral of Q₂ and because I(-θ,t)=-I(θ,t), Equation (48) becomes: 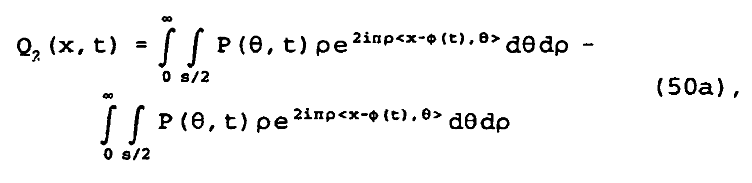 Hence, Equation (32) becomes: 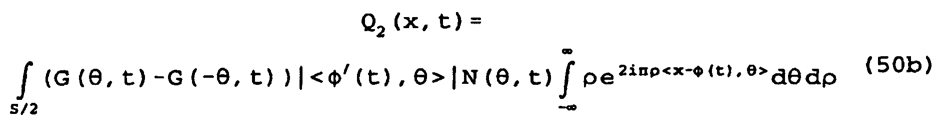 Integrating with respect to the variable p and replacing G(θ,t)-G(-θ,t) by the above-expression, one obtains: 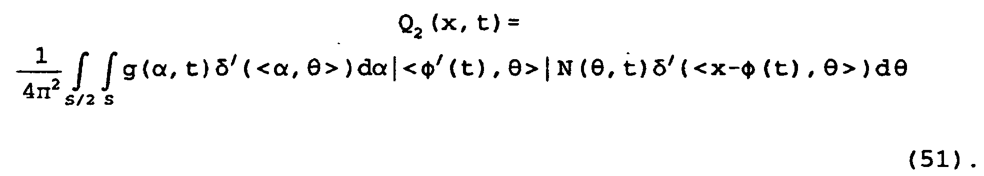 Equation (51) is similar to Equation (11) of the Defrise and Clack reference of record, but with a different weighting function N(θ,t). The following notation is used in transposing their results:
- (u,v): is the local coordinates of the intersection of the local coordinate plane (P₀) with the line going through φ(t) and parallel to the direction α;
- (uₓ, vₓ): is the local coordinates of the trace of the reconstruction point x;
- (s, ψ): is the polar parameters of the intersection of the local coordinate plane (P₀) with the plane perpendicular to the direction θ and going through x, and φ(t); and,
- LHₜ(s,ψ): is the integral of the function Hₜ along the line defined by the polar parameter (s,ψ), where s is the distance from Ω to the line, and ψ is the angle between the u-axis and the line, see FIGURE 10.

With this notation, Q₂ can be carried out as follows : 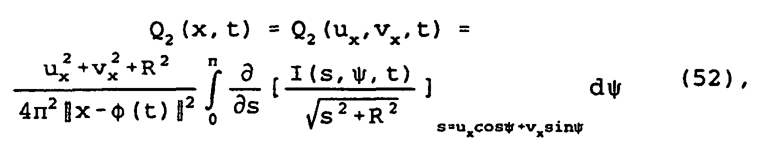 where: 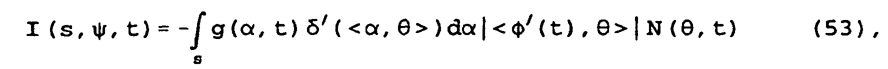 may be computed using Grangeat's fundamental relationship by multiplying |<φ'(t),θ>|N(θ,t) with the weighted partial derivatives of the line integrals of Hₜ: 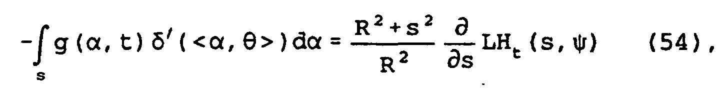 with Hₜ being a function defined on the local coordinate plane in a fashion similar to the one defined for computing Q₁, except for a constant factor:${\text{H}}_{\text{t}} \text{(u, v) =} \frac{\text{Rg (α, t)}}{\sqrt{{\text{R}}^{\text{2}} {\text{+ u}}^{\text{2}} {\text{+ v}}^{\text{2}}}}$

In other words, Q₂ is obtained by two-dimensionally backprojecting at the trace of the reconstruction point, partial derivatives of weighted partial derivatives of line integrals of weighted partial cone-beam data.

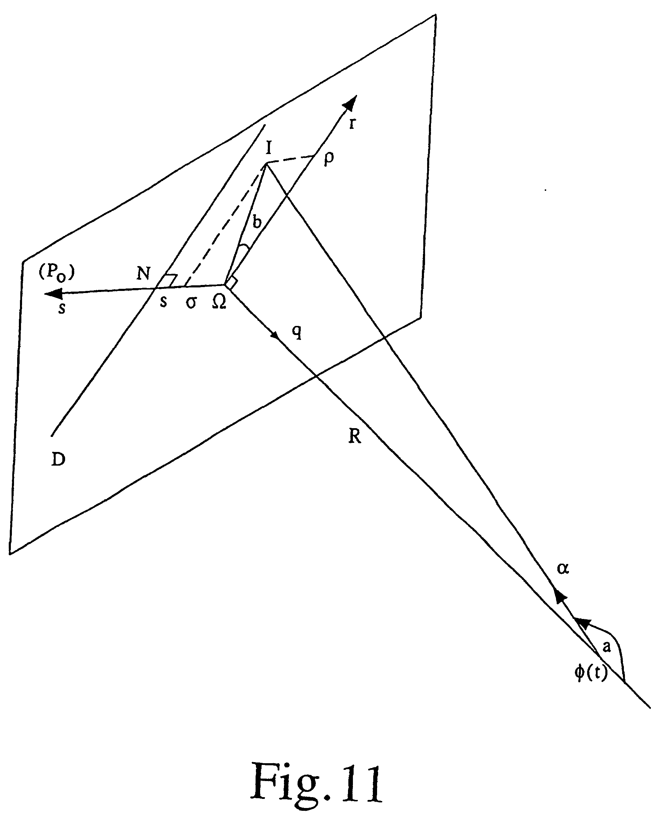

With reference to FIGURE 11, a coordinate system Ωqrs is defined with Ωq along Ωφ(t), Ωr parallel to the intersection line D of the color coordinate plane with the plane perpendicular to the unit vector θ, and Ωs perpendicular to D. In this coordinate system, the coordinates of the vector α are (ρ,σ,R)/(ρ²+σ²+R²)^{1/2} and those of the intersection point N of the line D with the axis Ωs are (0,s,0). Because the vector θ is perpendicular to both vectors (0,s,0) and the vector Nφ(t) with coordinates (0,-s,R), it follows that the coordinates of θ are proportional to the exterior product of the two vectors. Consequently, the coordinates of θ are (0,R,-s)/(s²+R²)^{1/2}, and hence:$\text{<α,θ> =} \frac{\text{R (σ-s)}}{\sqrt{{\text{ρ}}^{\text{2}} {\text{+ σ}}^{\text{2}} {\text{+ R}}^{\text{2}}} \sqrt{{\text{s}}^{\text{2}} {\text{+ R}}^{\text{2}}}}$ If a and b are the polar and azimuthal angles of the unit vectors α, see FIGURE 11, then:$\text{dα = sin (a) dadb}$ Because:$\text{cos (a) =} \frac{\text{-R}}{\sqrt{{\text{ρ}}^{\text{2}} {\text{+ σ}}^{\text{2}} {\text{+ R}}^{\text{2}}}}$ and,${\text{b = tan}}^{\text{-1}} \text{(} \frac{\text{σ}}{\text{ρ}} \text{)}$ it follows that the Jacobian is:$\frac{\text{∂(cos(a), b)}}{\text{∂(ρ, σ)}} {\text{= R(ρ}}^{\text{2}} {\text{+ σ}}^{\text{2}} {\text{+ R}}^{\text{2}} {\text{)}}^{\text{-3/2}}$ Hence, Equation (54) becomes: 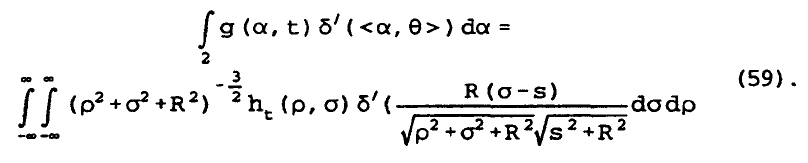 where hₜ(ρ,σ)=Rg(α,t). After integrating with respect to σ, this becomes:

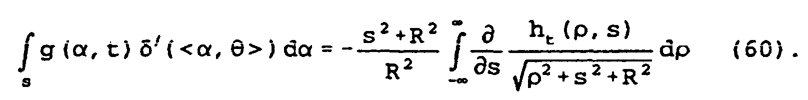

From this, a constructive form of Q₂ can be developed. In the local coordinate system in which integration is carried out with respect to θ over S/2, the vector θ can be obtained by multiplying its coordinates with respect to (r,s) coordinate system with the rotation matrix introduced by the angle (π/2-ψ). Consequently:$\text{θ =} \frac{\text{1}}{\sqrt{{\text{s}}^{\text{2}} {\text{+ R}}^{\text{2}}}} \text{(Rcosψ, Rsinψ, -s)}$ With respect to the local coordinate system:$\frac{\text{x-φ (t)}}{\left||\text{x-φ (t)}\right||} \text{=} \frac{\text{1}}{\sqrt{{\text{u}}_{\text{x}} {\text{}}^{\text{2}} {\text{+ v}}_{\text{x}} {\text{}}^{\text{2}} {\text{+ R}}^{\text{2}}}} {\text{(u}}_{\text{x}} {\text{,v}}_{\text{x}} \text{,R)}$ Consequently:$\text{<x-φ(t),θ> =} \frac{\text{R} \left||\text{x-φ (t)}\right||}{\sqrt{{\text{u}}_{\text{x}} {\text{}}^{\text{2}} {\text{+ v}}_{\text{x}} {\text{}}^{\text{2}} {\text{+ R}}^{\text{2}}} \sqrt{{\text{s}}^{\text{2}} {\text{+ R}}^{\text{2}}}} {\text{(u}}_{\text{x}} {\text{cosψ+v}}_{\text{x}} \text{sinψ-s)}$ Moreover, if a and b are the polar and the azimuthal angles of the vector θ, then:$\text{cos (a) =} \frac{\text{s}}{\sqrt{{\text{s}}^{\text{2}} {\text{+ R}}^{\text{2}}}}$ and$\text{b = ψ}$ Hence, the Jacobian is:$\frac{\text{∂ (cos (a), b)}}{\text{∂ (s, ψ)}} \text{=} \frac{{\text{R}}^{\text{2}}}{{\text{(s}}^{\text{2}} {\text{+ R}}^{\text{2}} {\text{)}}^{\text{3/2}}}$ Consequently: 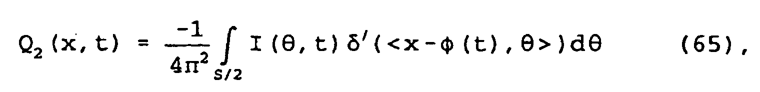 which becomes: 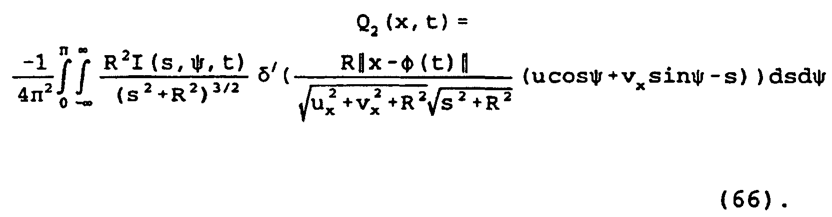 After integrating with respect to s:

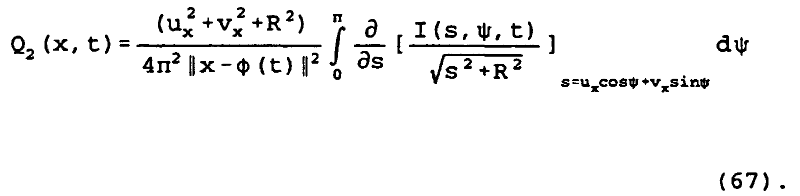

With particular reference to FIGURE 6, the Q₂ calculation **50** is divided into four parts. First, a processor or means **60** computes line integrals of the weighted partial cone-beam data. This is followed by a processor or means **62** for estimating the partial derivative of the line integrals with respect to the variable s, which represents the distance from the origin Ω of the local coordinate system to the lines in accordance with Equation (54). The partial derivatives of the line integrals Hₜ(s,ψ) are weighted by (R²+s²)/(4π²s²). This weighting is inversely proportional to cos²(a), where a denotes the divergence angle between the line φ(t)Ω and the plane generated by the vertex φ(t) and the line parameterized by (s,ψ). For simplicity of computation, the parallel beam geometry described by the parameters (s,ψ) on the local coordinate plane is utilized throughout the computation of Q₂.

A second part **46** computes the weight W associated with each line (s,ψ) in order to insure the normalization of the 3D backprojection when reconstructing the images. To insure this normalization, the number of intersections of a plane with the portion of the helix in which the data are three-dimensionally backprojected for reconstruction are computed for each plane defined by (s,ψ). A processor or means **70** identifies the affected volume Vₜ. More specifically, a processor or means **72** computes the traces A_{z} and B_{z} of the end points of the backprojection range for each slice, indexed by c, in the affected volume Vₜ. A processor or means **74** computes the weighting function Wₜ for each view t of each slice z in accordance with:${\text{W}}_{\text{tz}} {\text{(s, ψ) = N}}_{\text{tz}} \text{(s, ψ) |<φ'(t), (s, ψ) >|}$ Due to the fact that the truncated data is not used in backprojection, a count of the number of intersection vertices with non-truncated data along the plane is also counted.

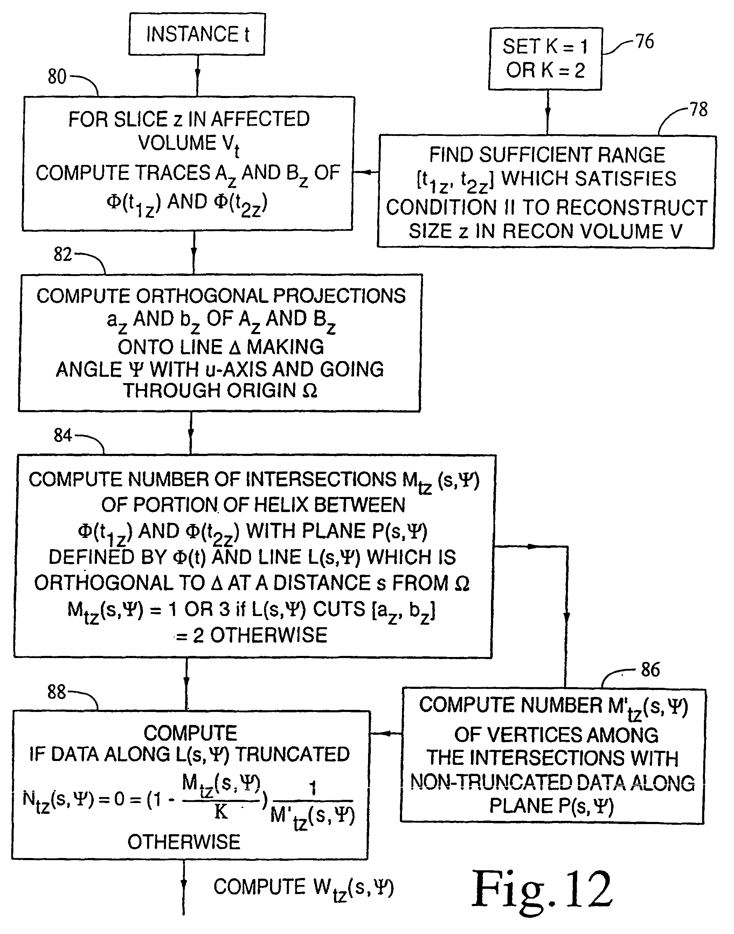

With reference to FIGURE 12 and continuing reference to FIGURE 6, without counting the current vertex φ(t), the number of intersections of a plane defined by the line (s,ψ) is equal to the number of intersections of the line (s,ψ) with the two branches of the trace of the portion of the helix in which the data are backprojected to create a slice of the reconstructed volume. This number is constant and equal to 1 when the line (s,ψ) cuts the line Δ outside the line segment defined by the orthogonal projections of Δ of the traces of the two extreme vertices of the portion of the helix. Inside this line segment, the number is either 0 or 2. That is, the line (s,ψ) either intersects the two branches of the trace or it does not have a common point with the trace at all. Second, because the weight is null for lines along which the data are truncated, it is not necessary to do any computation of line integrals or other related factors of these truncated lines.

More specifically, it is predetermined into how many slices the data is to be reconstructed, the number of pixels per slice, and the like. For each view t, a determination is made which slices are affected by view t, K is set **76** accordingly to either 1 or 2 for the reconstruction process. Once K is set, C from Equation (27) is known as is the weight of Q₁, which gives the range of each slice. At **78** a sufficient range [t_{1z}, t_{2z}] which satisfies the condition 2, i.e., that the angle between the half lines Mu' and Md' is less than the angle between Mu' and MI₁ to reconstruct slice C in the reconstruction volume V. From the range, the weighting for Q₂ is determined. For each view t, a step or means **80** computes the traces A_{z} and B_{z} of φ(t_{1z}) and φ(t_{2z}) for each slice z in the affected volume vₜ. A step or means **82** computes orthogonal projections a_{z} and b_{z} of the traces A_{z} and B_{z} onto the line Δ which makes an angle ψ with the u-axis and goes through the origin Ω.

With reference to FIGURES 6 and 12, a step or means **84** computes the number of intersections M_{tz}(s,ψ) of the portion of the helix between ψ(t_{1z}) and ψ(t_{2z}) with the plane P(s,ψ) defined by ψ(t) and the line L(s,ψ), which is orthogonal to the line Δ at a distance s from Ω. Again, the helix can only intersect the plane at 1, 2, or 3 points. M_{tz}(s,ψ)=1 or 3, if the line L of (s,ψ) cuts [a_{z},b_{z}]. Otherwise, M_{tz}(s,ψ)=2. The step or means **86** computes the number M'_{tz}(s,ψ) of the vertices among the intersections with non-truncated data along the plane P(s,ψ). A step or means **88** computes the number N_{tz}(s,ψ) which is supplied to the step or means **74** to compute the weighting function W_{tz}(s,ψ). The number of intersections
is calculated in accordance with:${\text{N}}_{\text{tz}} \text{(s,ψ) = 0}$ if data along L(s,ψ) is truncated, and${\text{N}}_{\text{tz}} \text{(s, ψ) = (1-} \frac{{\text{M}}_{\text{tz}} \text{(s, ψ)}}{\text{K}} \text{)} \frac{\text{1}}{{\text{M}}_{\text{tz}}^{\text{'}} \text{(s, ψ)}}$ otherwise.

With reference again to FIGURE 6, a processor **90** determines a partial derivative with respect to s and weights it. More specifically, a step or means **92** computes I_{tz}(s,ψ)=W_{tz}(s,ψ)Pₜ(s,ψ). A step or means **94** determines the Jacobian J_{tz}(s,ψ) as follows:${\text{J}}_{\text{tz}} \text{(s, ψ) =} \frac{\text{∂}}{\text{∂s}} \frac{{\text{I}}_{\text{tz}} \text{(s, ψ)}}{\sqrt{{\text{s}}^{\text{2}} {\text{+ R}}^{\text{2}}}}$

A processor **96** multiplies, at each pixel (u,v) in the local coordinate plane, the backprojection of (u,v) from the processor **90** with the square of the distance from the vertex φ(t) to the pixel. More specifically, a step or means **98** adds up 180° of parallel beam backprojections. It performs a parallel beam two-dimensional backprojection of J_{tz} at each (u,v) to compute B_{tz}(u,v). A step or means **100** computes Q_{2tz}(u,v)=(u²+v²+R²)B_{tz}(u,v). These values of Q₂ are supplied to the summation means **52** to be combined with the corresponding values of Q₁.

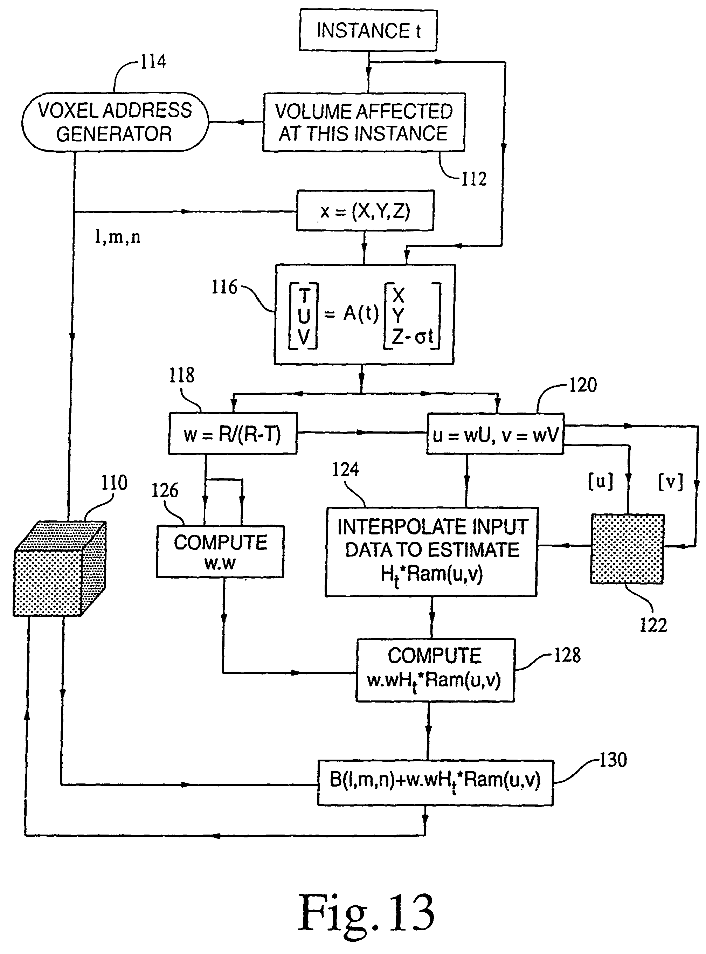

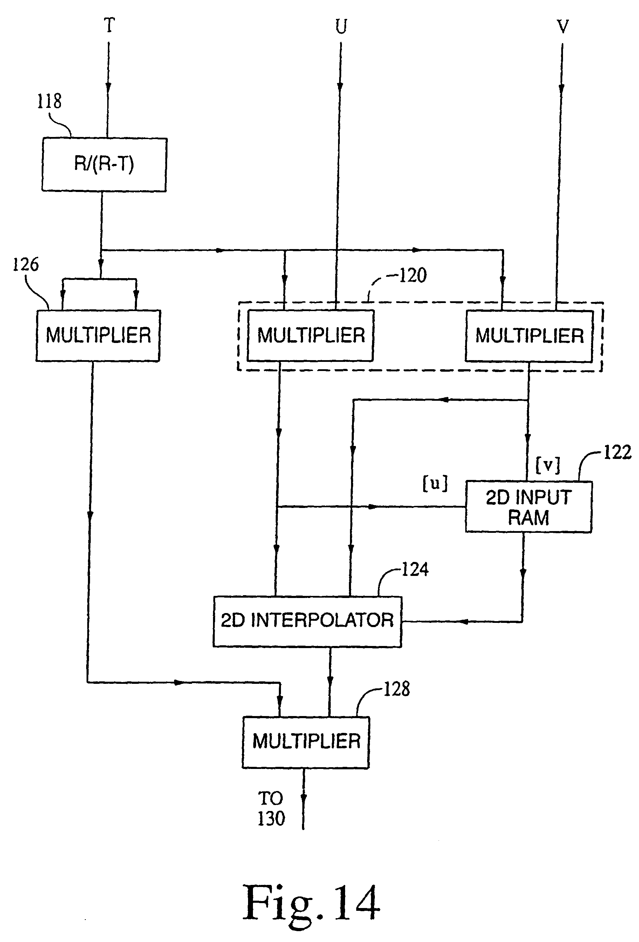

With reference to FIGURES 13 and 14, the three-dimensional backprojector **54** is a processor which computes the final reconstruction from the processed data, such as the above-described convolved data. The backprojector is an estimator of: 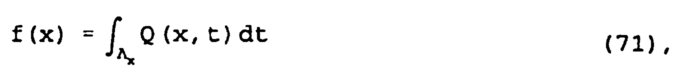 where Q represents the processed data. Because the data are collected at only a finite number of instances, the integral can be approximated by the finite sum:${\text{f(x) = ΔtΣQ(x,t}}_{\text{i}} \text{)}$ Further, the continuous volume can be represented by a finite number of voxels of size ΔXΔYΔZ, that is x= (lΔX, mΔY, nΔZ)+(X₀,Y₀,Z₀) for some 1=0, 1, ..., L-1, m=0, 1, ... , M-1, n=0, 1, ..., N-1. (X₀,Y₀,Z₀) is a corner of the represented volume.

When Q is reduced to Q₁, then Q is estimated by appropriately weighting the convolved data Hₜ. Due to the fact that at each instance t, data are collected only along a finite number of rays within a partial cone-beam, Hₜ(u,v) must be approximated from a sample {Hₜ(iΔu+u₀, jΔv+v₀) | i=0, 1, ... , I-1, and j=0, 1, ..., J-1}. It should further be noted that because there is data within a partial cone geometry, the backprojection of the processed data does not affect the whole represented volume, but only a portion of it. Another important point is that the backprojection is conveniently carried out in a new coordinate system defined by (C(t),U,V).

With particular reference to FIGURE 13, a three-dimensional image memory **110** receives the backprojected views. As each view t is received, an affected volume determining means or step **112** determines the part of the volume of memory **110** which is affected by the processed view t. A voxel generator **114** sweeps through all of the voxels in the affected part of the volume. A coordinate transform processor **116** converts the absolute coordinate system (X,Y,Z) of the voxel address generator into the local coordinate system (T,U,V). With continuing reference to FIGURE 13 and further reference to FIGURE 14, a weight generating system includes a first weight generator **118** which generates a first weighting value w which is equal to R/(R-T). A two-dimensional input generator **120** which generates local coordinates of a voxel given by the voxel generator, the local address being (u,v) and generates weighting factors u and w, where u=wU and v=wV. The u and v addresses address a two-dimensional memory **122** in which the two-dimensional view Hₜ*RAM is stored. An input interpolator **124** uses the input data retrieved from the input memory **122** and fractional parts of the new coordinates calculated by the two-dimensional input generator **120** to estimate the input data corresponding to a ray going through the voxel given by the voxel generator. A multiplier **126** multiplies the weighting factor w by itself to form a weighting factor w². A multiplier **128** multiplies the interpolated input data Hₜ*RAM(u,v) by the squared weighting function w². An adder **130** retrieves the current values B(l,m,n) retrieved from the volumetric memory **110** with the product from the multiplier **128**. In this manner, the values stored in each voxel is updated with each two-dimensional set of processed data. Initially, the three-dimensional output memory **110** has each pixel value assigned a nominal, non-zero starting value, e.g., 1. In this manner, the value at each voxel is iteratively updated until an accurate reconstruction is obtained.

## Claims

1. A method of diagnostic imaging, including the steps of
• moving a vertex of a cone-beam of radiation along a helical trajectory,
• irradiating a subject with the cone-beam of radiation,
• converting the passed radiation into a plurality of partial cone-beam views, whereby the partial cone-beam views are untruncated in a direction parallel to a tangent to the trajectory at the vertex and are truncated in another direction, and
• reconstructing the partial cone-beam views into a volumetric image representation, the reconstruction including the steps of
o processing each of the partial cone-beam views to create a weighted view,
o computing a first two-dimensional component (Q1), which is proportional to a constant part of a redundancy weight function, essentially by one-dimensionally convolving the weighted view along the direction of the tangent to the helical trajectory at the vertex with a reconstruction filter,
o computing a second two-dimensional component (Q2), which is corresponding to a non-constant part of the redundancy weight function, essentially by two-dimensionally backprojecting at the trace of the reconstruction point a partial derivative of a weighted partial derivative of line integrals of the weighted view, the weighted view being multiplied with the non-constant part of the redundancy weight function, the non-constant part of the redundancy weight function being computed on basis of intersection points of a plane with a portion of the helical trajectory, and
o backprojecting the first component and the second component into an affected portion of the volumetric image representation.

2. A method as claimed in claim 1 further comprising the steps of:
• defining planes and voxel locations of the volumetric image representation; and
• reconstructing the partial cone-beam views based on the defined planes and voxel locations such that the partial cone-beam views contribute to at least two of the planes to generate the volumetric image representation.

3. A method as claimed in claim 1, wherein the steps include:
• receiving the radiation with a radiation detector having a two-dimensional array of detector elements;
• rotating the vertex along at least an helical arc segment of the helical trajectory;
• sampling the radiation detector at a plurality of angular increments along the helical arc segment to generate a plurality of two-dimensional data value arrays, each of the data values corresponding to a detector element and each of the arrays corresponding to a partial cone-beam view;
and the reconstructing steps include:
• weighting the data values of each two-dimensional array in accordance with a cosine of an angle between a ray originating from the vertex and corresponding to the detector elements and a ray originating from the vertex and being normal to a local coordinate plane at the radiation detector;
• multiplying each value of the second components in accordance with a square of a distance from the vertex to the detector element corresponding to the value of the second component;
• adding the first component and the second component; and
• three-dimensionally backprojecting the sum into a volumetric image memory.

4. A method as claimed in claim 1, wherein the reconstruction steps include:
• computing a cosine weighted partial derivative of the line integrals.

5. A method as claimed in claim 1 further comprising the step of computing a weighting function, which includes:
• determining a sub-volume of the volumetric image representation affected by each of the partial cone-beam views; and
• computing traces of end points of a backprojection range for each slice within the volumetric image representation, and a means for multiplying by a weighting value based on the trace to generate the weighting function.

6. A method as claimed in claim 5 further comprising the step of generating the weighting value, which includes:
• computing orthogonal projections of the traces;
• computing a number of the intersection points between the portion of the helical trajectory and the plane that is defined by the partial derivative of the line integrals;
• determining a number of times that the plane intersects the portion of the helical trajectory;
• determining intersection points of vertices with non-truncated data along the plane; and
• setting the weighting function to zero for truncated data and for setting the weighting function equal to a value which is proportional to 1 minus the number of intersection points divided by a constant and weighted by an inverse of a number of vertices among the intersection points with non-truncated data to generate the weighting value.

7. A method as claimed in claim 2, wherein the reconstructing steps include backprojecting each of the sums into a three-dimensional volumetric region of the volumetric image representation such that each backprojected sum contributes to a plurality of parallel planes of the volumetric image representation.

8. A method as claimed in claim 7, wherein the backprojecting step includes:
• determining a volumetric segment of the volumetric image representation affected by each two-dimensional backprojected data set;
• generating voxel addresses for the three-dimensional volumetric image representation corresponding to the affected volume;
• coordinating a coordinate system of the volumetric image representation and each two-dimensional data set;
• addressing the two-dimensional data set to retrieve data values corresponding to each voxel address generated;
• weighting each retrieved data value from the two-dimensional data set; and
• retrieving a stored data value from the volumetric image representation corresponding to each voxel address generated and adding the corresponding weighted data value from the two-dimensional data set thereto.

9. A diagnostic imaging apparatus that comprises
• a radiation source (16) provided for generating a cone-beam (20) of radiation,
• means provided for moving the radiation source and a subject relative to each other such that a vertex of the cone-beam (20) of radiation moves along a helical trajectory,
• a radiation detector (30) provided for converting radiation that has passed through the subject into a plurality of two-dimensional partial cone-beam views, each partial cone-beam view being untruncated in a direction parallel to a tangent to the helical trajectory at the vertex and being truncated in another direction, and
• reconstruction means (44, 46, 48, 50, 52, 54) provided for processing the partial cone-beam views and for backprojecting the processed partial cone-beam views into an volumetric image representation, the reconstruction means comprising
o processor means (44) provided for computing a weighted view for each of the partial cone-beam views,
o processor means (48) provided for computing a first two-dimensional component (Q1), which is proportional to a constant part of a redundancy weight function, essentially by one-dimensionally convolving the weighted view along the direction of the tangent to the helical trajectory at the vertex with a reconstruction filter,
o processor means (46, 50) provided for computing a second two-dimensional component (Q2), which is corresponding to a non-constant part of the redundancy weight function, essentially by two-dimensionally backprojecting at the trace of the reconstruction point a partial derivative of a weighted partial derivative of line integrals of the weighted view, the weighted view being multiplied with the non-constant part of the redundancy weight function, the non-constant part of the redundancy weight function being computed on basis of intersection points of a plane with a portion of the helical trajectory, and
o backprojector means (52, 54) for backprojecting the first component and the second component into an affected portion of the volumetric image representation.

10. Apparatus as claimed in claim 9, wherein the reconstruction means are arranged to reconstruct the partial cone-beam views based on defined planes and voxel locations of the volumetric image representation such that the partial cone-beam views contribute to multiple planes to generate the volumetric image representation.

11. Apparatus as claimed in one of claims 9 or 11, wherein the radiation detector is arranged
• to receive the radiation on an two-dimensional array of detector elements; and
• to being sampled at a plurality of angular increments along an helical arc segment of the helical trajectory moved by the vertex to generate a plurality of two-dimensional data value arrays, each of the data values corresponding to a detector element and each of the arrays corresponding to one of the partial cone-beam views;
and the reconstructing means being arranged
• to weight the data values of each two-dimensional array in accordance with a cosine of an angle between a ray originating from the vertex and corresponding to the detector element and a ray originating from the vertex and being normal to a local coordinate plane at the radiation detector;
• to multiply each value of the second components in accordance with a square of a distance from the vertex to the detector element corresponding to the data value;
• to add the first component and the second component,
and the backprojector means being arranged for three-dimensionally backprojecting the sum into a volumetric image memory.

12. Apparatus as claimed in claim 9, wherein the reconstruction means being arranged
• to compute a cosine weighted partial derivative of the line integrals.

13. Apparatus as claimed in claim 9, wherein the reconstruction means include processor means (70, 72, 74, 82, 84, 86, 88) to compute a weighting function and which processor means are being arranged
• to determine a sub-volume of the volumetric image representation affected by each of the partial cone-beam views; and
• to compute traces of end points of the backprojection range for each slice within the volumetric image representation, and a means for multiplying by a weighting value based on the trace to generate the weighting function.

14. Apparatus as claimed in claim 13, wherein the processor means (70, 72, 74, 82, 84, 86, 88) for computing the weighting value are further arranged
• to compute orthogonal projections of the traces;
• to compute a number of intersections between the helical arc segment and a plane defined by the first derivative of the line integrals;
• to determine a number of times that the plane intersects the helical arc segment;
• to determine intersections of vertices with non-truncated data along the plane; and
• to set the weighting function to zero for truncated data and to set the weighting function equal to a value which is proportional to 1 minus the number of intersections divided by a constant and weighted by an inverse of a number of vertices among the intersections with non-truncated data to generate the weighting value.

15. Apparatus as claimed in any one of claims 9 to 14, wherein the reconstruction processor is arranged to backproject each of the partial cone-beam views into a three-dimensional volumetric region of the volumetric image representation such that each backprojected view contributes to a plurality of parallel planes of the volumetric image representation.

16. Apparatus as claimed in claim 15, wherein the reconstruction processor is arranged
• to determine a volumetric segment of the volumetric image representation affected by each two-dimensional backprojected data set;
• to generate voxel addresses for the three-dimensional volumetric image representation corresponding to the affected volume;
• to coordinate a coordinate system of the volumetric image representation and each two-dimensional data set;
• to address the two-dimensional data set to retrieve data values corresponding to each voxel address generated;
• to weight each retrieved data value from the two-dimensional data set; and
• to retrieve a stored data value from the volumetric image representation corresponding to each voxel address generated and adding the corresponding weighted data value from the two-dimensional data set thereto.

## Patentansprüche

1. Verfahren zur diagnostischen Bildgebung, das die folgenden Schritte umfasst:
• Bewegen des Scheitelpunkt eines Kegelstrahls entlang einer spiralförmigen Bahn,
• Bestrahlen einer Person mit dem Kegelstrahl,
• Konvertieren der hindurchgegangenen Strahlung in eine Vielzahl von Teilkegelstrahlansichten, wobei die Teilkegelstrahlansichten in einer Richtung parallel zu einer Bahntangente am Scheitelpunkt nicht abgeschnitten werden und in einer anderen Richtung abgeschnitten werden, und
• Rekonstruieren der Teilkegelstrahlansichten zu einer volumetrischen Bilddarstellung, wobei das Rekonstruktieren die folgenden Schritte umfasst:
o Verarbeiten jeder Teilkegelstrahlansicht, um eine gewichtete Ansicht zu erzeugen,
o Berechnen einer ersten zweidimensionalen Komponenten (Q1), die sich proportional zu einem konstanten Teil einer Redundanzgewichtungsfunktion verhält, indem im Wesentlichen eine eindimensionale Faltung der gewichteten Ansicht entlang der Richtung der Tangente zur spiralförmigen Bahn am Scheitelpunkt mit einem Rekonstruktionsfilter vorgenommen wird,
o Berechnen einer zweiten zweidimensionalen Komponenten (Q2), die einem nicht konstanten Teil der Redundanzgewichtungsfunktion entspricht, indem im Wesentlichen an der Spur des Rekonstruktionspunktes eine zweidimensionale Rückprojektion einer partiellen Ableitung einer gewichteten partiellen Ableitung von Linienintegralen der gewichteten Ansicht vorgenommen wird, wobei die gewichtete Ansicht mit dem nicht konstanten Teil der Redundanzgewichtungsfunktion multipliziert wird, der auf der Basis der Schnittpunkte einer Ebene mit einem Teil der spiralförmigen Bahn berechnet wurde, und
o Rückprojizieren der ersten und der zweiten Komponente in einen betroffenen Bereich der volumetrischen Bilddarstellung:

2. Verfahren nach Anspruch 1, das weiterhin folgende Schritte umfasst:
• Definieren von Ebenen und Voxelpositionen der volumetrischen Bilddarstellung; und
• Rekonstruieren der Teilkegelstrahlansichten basierend auf den definierten Ebenen und Voxelpositionen, so dass die Teilkegelstrahlansichten zu mindestens zwei der Ebenen beitragen, um die volumetrische Bilddarstellung zu erzeugen.

3. Verfahren nach Anspruch 1, wobei die Schritte Folgendes umfassen:
• Empfangen der Strahlung mit einem Strahlungsdetektor, der eine zweidimensionale Anordnung von Detektorelementen aufweist;
• Drehen des Scheitelpunktes entlang mindestens eines spiralförmigen Bogensegments der spiralförmigen Bahn;
• Abtasten des Strahlungsdetektoren an einer Vielzahl von Winkelinkrementen entlang des spiralförmigen Bogensegments, um eine Vielzahl von zweidimensionalen Datenwertanordnungen zu erzeugen, wobei jeder der Datenwerte einem Detektorelement und jede der Anordnungen einer Teilkegelstrahlansicht entspricht;
und die Rekonstruktionsschritte Folgendes umfassen:
• Gewichten der Datenwerte jeder zweidimensionalen Anordnung gemäß dem Kosinus eines Winkels zwischen einem Strahl, der vom Scheitelpunkt ausgeht und den Detektorelementen entspricht, und einem Strahl, der vom Scheitelpunkt ausgeht und senkrecht zu einer lokalen Koordinatenebene am Strahlungsdetektor verläuft;
• Multiplizieren jedes Wertes der zweiten Komponenten gemäß dem Quadrat eines Abstandes vom Scheitelpunkt zum Detektorelement, das dem Wert der zweiten Komponente entspricht;
• Addieren der ersten Komponente und der zweiten Komponente; und
• dreidimensionales Rückprojizieren der Summe in einen volumetrischen Bildspeicher.

4. Verfahren nach Anspruch 1, wobei die Rekonstruktionsschritte, Folgendes umfassen:
• Berechnen einer kosinus-gewichteten partiellen Ableitung der Linienintegrale.

5. Verfahren nach Anspruch 1, das weiterhin den Schritt der Berechnung einer Gewichtungsfunktion beinhaltet, der Folgendes umfasst:
• Bestimmen eines Teilvolumens der von jeder der Teilkegelstrahlansichten betroffenen volumetrischen Bilddarstellung; und
• Berechnen von Endpunktspuren eines Rückprojektionsbereiches für jede Schicht innerhalb der volumetrischen Bilddarstellung, und ein Mittel zum Multiplizieren mit einem auf der Spur basierenden Gewichtungswert, um die Gewichtungsfunktion zu erzeugen.

6. Verfahren nach Anspruch 5, das weiterhin den Schritte der Erzeugung des Gewichtungswertes beinhaltet, der Folgendes umfasst:
• Berechnen orthogonaler Projektionen der Spuren;
• Berechnen einer Anzahl von Schnittpunkten zwischen dem Abschnitt der spiralförmigen Bahn und der Ebene, der durch die partielle Ableitung der Linientintegrale definiert wird;
• Bestimmen der Anzahl von Malen, die die Ebene den Abschnitt der spiralförmigen Bahn schneidet;
• Bestimmen der Schnittpunkte von Scheitelpunkten mit nicht abgeschnittenen Daten entlang der Ebene; und
• Nullsetzen der Gewichtungsfunktion für abgeschnittene Daten und Gleichsetzen der Gewichtungsfunktion für nicht abgeschnittene Daten mit einem Wert, der proportional zu 1 minus der Anzahl der Schnittpunkte geteilt durch eine Konstante und mit einem Kehrwert einer Anzahl von Scheitelpunkten unter den Schnittpunkten gewichtet ist, um den Gewichtungswert zu erzeugen.

7. Verfahren nach Anspruch 2, wobei die Rekonstruktionsschritte das Rückprojizieren von jeder der Summen in einen dreidimensionalen volumetrischen Bereich der volumetrischen Bilddarstellung umfassen, so dass jede rückprojizierte Summe zu einer Vielzahl paralleler Ebenen der volumetrischen Bilddarstellung beiträgt.

8. Verfahren nach Anspruch 7, wobei der Schritt der Rückprojektion Folgendes umfasst:
• Bestimmen eines volumetrischen Segments der von jedem zweidimensionalen Rückprojektionsdatensatz betroffenen volumetrischen Bilddarstellung;
• Erzeugen von Voxeladressen für die dreidimensionale volumetrische Bilddarstellung, die dem betroffenen Volumen entsprechen;
• Koordinieren eines Koordinatensystems der volumetrischen Bilddarstellung und jedes zweidimensionalen Datensatzes;
• Adressieren des zweidimensionalen Datensatzes, um Datenwerte abzurufen, die jeder erzeugten Voxeladresse entsprechen;
• Gewichten jedes aus dem zweidimensionalen Datensatz abgerufenen Datensatzes; und
• Abrufen eines gespeicherten Datenwertes aus der volumetrischen Bilddarstellung, der jeder erzeugten Voxeladresse entspricht, und Addieren des entsprechenden gewichteten Datenwertes aus dem zweidimensionalen Datensatz hierzu.

9. Diagnostische Bildgebungsvorrichtung, die Folgendes umfasst:
• eine Strahlungsquelle (16) zur Erzeugung eines Kegelstrahls (20),
• Mittel, um die Strahlungsquelle und eine Person relativ zueinander zu bewegen, so dass sich ein Scheitelpunkt des Kegelstrahls (20) entlang einer spiralförmigen Bahn bewegt,
• ein Strahlungsdetektor (30), um Strahlung, die die Person durchquert hat, in eine Vielzahl von zweidimensionalen Teilkegelstrahlansichten umzuwandeln, wobei jede Teilkegelstrahlansicht in einer Richtung parallel zu einer Tangente der spiralförmigen Bahn am Scheitelpunkt nicht abgeschnitten ist und in einer anderen Richtung abgeschnitten ist, und
• Rekonstruktionsmittel (44, 46, 48, 50, 52, 54), um die Teilkegelstrahlansichten zu verarbeiten und die verarbeiteten Teilkegelstrahlansichten in eine volumetrische Bilddarstellung rückzuprojizieren, wobei die Rekonstruktionsmittel Folgendes umfassen:
o Prozessormittel (44), um für jede der Teilkegelstrahlansichten eine gewichtete Ansicht zu berechnen,
o Prozessormittel (48), um eine erste zweidimensionale Komponente (Q1) zu berechnen, die sich proportional zu einem konstanten Teil einer Redundanzgewichtungsfunktion verhält, indem im Wesentlichen eine eindimensionale Faltung der gewichteten Ansicht entlang der Richtung der Tangente zur spiralförmigen Bahn am Scheitelpunkt mit einem Rekonstruktionsfilter vorgenommen wird,
o Prozessormittel (46, 50), um eine zweite zweidimensionale Komponente (Q2) zu berechnen, die einem nicht konstanten Teil der Redundanzgewichtungsfunktion entspricht, indem im Wesentlichen an der Spur des Rekonstruktionspunktes eine zweidimensionale Rückprojektion einer partiellen Ableitung einer gewichteten partiellen Ableitung von Linienintegralen der gewichteten Ansicht vorgenommen wird, wobei die gewichtete Ansicht mit dem nicht konstanten Teil der Redundanzgewichtungsfunktion multipliziert wird, der auf der Basis der Schnittpunkte einer Ebene mit einem Abschnitt der spiralförmigen Bahn berechnet wird, und
o Rückprojektionsmittel (52, 54), um die erste und die zweite Komponente in einen betroffenen Bereich der volumetrischen Bilddarstellung rückzuprojizieren.

10. Vorrichtung nach Anspruch 9, wobei die Rückprojektionsmittel vorgesehen sind, um die Teilkegelstrahlansichten basierend auf definierten Ebenen und Voxelpositionen so zu rekonstruieren, dass die Teilkegelstrahlansichten zu mehreren Ebenen beitragen, um die volumetrische Bilddarstellung zu erzeugen.

11. Vorrichtung nach Anspruch 9 oder 10, wobei der Strahlungsdetektor vorgesehen ist,
• um die Strahlung auf einer zweidimensionalen Anordnung von Detektorelementen zu empfangen, und
• um an einer Vielzahl von Winkelinkrementen entlang eines spiralförmigen Bogensegments der vom Scheitelpunkt zurückgelegten spiralförmigen Bahn abgetastet zu werden, um eine Vielzahl von zweidimensionalen Datenwertanordnungen zu erzeugen, wobei jeder der Datenwerte einem Detektorelement und jede der Anordnungen einer der Teilkegelstrahlansichten entspricht;
und die Rückprojektionsmittel vorgesehen sind,
• um die Datenwerte jeder zweidimensionalen Anordnung gemäß dem Kosinus eines Winkels zwischen einem Strahl, der vom Scheitelpunkt ausgeht und den Detektorelementen entspricht, und einem Strahl, der vom Scheitelpunkt ausgeht und senkrecht zu einer lokalen Koordinatenebene am Strahlungsdetektor verläuft, zu gewichten;
• um jeden Wert der zweiten Komponenten gemäß dem Quadrat eines Abstandes vom Scheitelpunkt zum Detektorelement, das dem Datenwert entspricht, zu multiplizieren;
• um die erste Komponente und die zweite Komponente zu addieren,
und die Rückprojektionsmittel für eine dreidimensionale Rückprojektion der Summe in einen volumetrischen Bildspeicher vorgesehen sind.

12. Vorrichtung nach Anspruch 9, wobei die Rückprojektionsmittel vorgesehen sind
• um eine kosinus-gewichtete partielle Ableitung der Linienintegrale zu berechnen.

13. Vorrichtung nach Anspruch 9, wobei die Rückprojektionsmittel Prozessormittel (70, 72, 74, 82, 84, 86, 88) umfassen, um eine Gewichtungsfunktion zu berechnen, und die Prozessormittel vorgesehen sind
• um ein Teilvolumen der von jeder der Teilkegelstrahlansichten betroffenen volumetrischen Bilddarstellung zu bestimmen; und
• Endpunktspuren des Rückprojektionsbereiches für jede Schicht innerhalb der volumetrischen Bilddarstellung zu berechnen, und ein Mittel enthalten, um einen auf der Spur basierenden Gewichtungswert zu multiplizieren und die Gewichtungsfunktion zu erzeugen.

14. Vorrichtung nach Anspruch 13, wobei die Prozessormittel (70, 72, 74, 82, 84, 86, 88) für die Berechnung des Gewichtungswertes weiterhin vorgesehen sind
• um orthogonale Projektionen der Spuren zu berechnen;
• um eine Anzahl von Schnittpunkten zwischen dem spiralförmigen Bogensegment und einer Ebene zu berechnen, die durch erste Ableitung der Linientintegrale definiert wird;
• um eine Anzahl von Malen, die die Ebene den Teil das spiralförmigen Bogensegment schneidet, zu bestimmen;
• um Schnittpunkte der Scheitelpunkte mit den nicht abgeschnittenen Daten entlang der Ebene zu bestimmen; und
• um die Gewichtungsfunktion für abgeschnittene Daten auf Null zu setzen, und die Gewichtungsfunktion für nicht abgeschnittene Daten mit einem Wert gleich zu setzen, der proportional zu 1 minus der Anzahl der Schnittpunkte geteilt durch eine Konstante und mit einem Kehrwert einer Anzahl von Scheitelpunkten unter den Schnittpunkten gewichtet ist, um den Gewichtungswert zu erzeugen.

15. Vorrichtung nach einem der Ansprüche 9 bis 14, wobei der Rekonstruktionsprozessor vorgesehen ist, um jede der Teilkegelstrahlansichten in einen dreidimensionalen volumetrischen Bereich der volumetrischen Bilddarstellung rückzuprojizieren, so dass jede rückprojizierte Summe zu einer Vielzahl paralleler Ebenen der volumetrischen Bilddarstellung beiträgt.

16. Vorrichtung nach Anspruch 15, wobei der Rekonstruktionsprozessor vorgesehen ist
• um ein volumetrisches Segment der von jedem zweidimensionalen Rückprojektionsdatensatz betroffenen volumetrischen Bilddarstellung zu bestimmen;
• um Voxeladressen für die dreidimensionale volumetrische Bilddarstellung zu erzeugen, die dem betroffenen Volumen entsprechen;
• um ein Koordinatensystem der volumetrischen Bilddarstellung und jeden zweidimensionalen Datensatz zu koordinieren;
• um den zweidimensionalen Datensatz zu adressieren, um Datenwerte abzurufen, die jeder erzeugten Voxeladresse entsprechen;
• um jeden aus dem zweidimensionalen Datensatz abgerufenen Datensatz zu gewichten; und
• um einen gespeicherten Datenwert aus der volumetrischen Bilddarstellung abzurufen, der jeder erzeugten Voxeladresse entspricht, und um den entsprechenden gewichteten Datenwert aus dem zweidimensionalen Datensatz hierzu zu addieren.

## Revendications

1. Procédé d'imagerie diagnostique comprenant les étapes suivantes :
- le déplacement d'un sommet d'un faisceau conique de rayonnement le long d'une trajectoire en spirale,
- l'irradiation d'un sujet par le faisceau conique de rayonnement,
- la conversion du rayonnement envoyé en une pluralité de vues de faisceaux coniques partiels de sorte que les vues de faisceaux coniques partiels ne soient pas tronquées dans une direction parallèle à une tangente à la trajectoire au sommet et soient tronquées dans une autre direction, et
- la reconstruction des vues de faisceaux coniques partiels en une représentation d'image volumétrique, la reconstruction comprenant les étapes suivantes :
* le traitement de chacune des vues de faisceaux coniques partiels pour créer une vue pondérée,
* le calcul d'une première composante bidimensionnelle (Q1), qui est proportionnelle à une partie constante d'une fonction de pondération redondante, essentiellement par convolution unidimensionnelle de la vue pondérée le long de la direction de la tangente à la trajectoire en spirale au sommet avec un filtre de reconstruction,
* le calcul d'une seconde composante bidimensionnelle (Q2) qui correspond à une partie non constante de la fonction de pondération redondante, essentiellement en rétroprojetant bidimensionnellement, sur la trace du point de reconstruction, une dérivée partielle d'une dérivée partielle pondérée d'intégrales linéaires de la vue pondérée, la vue pondérée étant multipliée par la partie non constante de la fonction de pondération redondante, la partie non constante de la fonction de pondération redondante étant calculée sur la base de points d'intersection d'un plan avec une partie de la trajectoire en spirale, et
* la rétroprojection de la première composante et de la seconde composante dans une partie affectée de la représentation d'image volumétrique.

2. Procédé selon la revendication 1 comprenant en outre les étapes suivantes :
- la définition de plans et d'emplacements de voxels de la représentation d'image volumétrique; et
- la reconstruction des vues de faisceaux coniques partiels sur la base des plans et des emplacements de voxels définis de sorte que les vues de faisceaux coniques partiels contribuent à au moins deux des plans pour générer la représentation d'image volumétrique.

3. Procédé selon la revendication 1 dans lequel les étapes comprennent :
- la réception du rayonnement par un détecteur de rayonnement ayant une matrice bidimensionnelle d'éléments détecteurs;
- la rotation du sommet le long d'au moins un segment d'arc spiralé de la trajectoire spiralée;
- l'échantillonnage du détecteur de rayonnement dans une pluralité d'incréments angulaires le long du segment d'arc spiralé pour générer une pluralité de matrices de valeurs de données bidimensionnelles, chacune des valeurs de données correspondant à un élément détecteur et chacune des matrices correspondant à une vue de faisceau conique partiel;
et les étapes de reconstruction comprennent :
- la pondération des valeurs de données de chaque matrice bidimensionnelle selon un cosinus d'un angle entre un rayon provenant du sommet et correspondant aux éléments détecteurs et un rayon provenant du sommet et qui est normal au plan de coordonnées locales au niveau du détecteur de rayonnement;
- la multiplication de chaque valeur des secondes composantes conformément à un carré de la distance entre le sommet et l'élément détecteur correspondant à la valeur de la seconde composante;
- l'addition de la première composante et de la seconde composante; et
- la rétroprojection tridimensionnelle de la somme dans une mémoire d'images volumétriques.

4. Procédé selon la revendication 1 dans lequel les étapes de reconstruction comprennent:
- le calcul d'une dérivée partielle pondérée cosinusoïdale des intégrales linéaires.

5. Procédé selon la revendication 1 comprenant en outre l'étape de calcul d'une fonction de pondération qui comprend :
- la détermination d'un sous-volume de la représentation d'image volumétrique affectée par chacune des vues de faisceaux coniques partiels; et
- le calcul de traces de points terminaux d'une plage de rétroprojection pour chaque tranche de la représentation d'image volumétrique, et un moyen pour multiplier par une-valeur de pondération basée sur la trace pour générer la fonction de pondération.

6. Procédé selon la revendication 5 comprenant en outre l'étape de génération de la valeur de pondération qui comprend :
- le calcul de projections orthogonales des traces;
- le calcul d'un certain nombre de points d'intersection entre la partie de la trajectoire spiralée et le plan qui est défini par la dérivée partielle des intégrales linéaires;
- la détermination du nombre de fois que le plan coupe la partie de la trajectoire spiralée;
- la détermination de points d'intersection de sommets avec des données non tronquées le long du plan; et
- le réglage de la fonction de pondération sur zéro pour les données tronquées et pour régler la fonction de pondération égale à une valeur qui est proportionnelle à 1 moins le nombre de points d'intersection divisée par une constante et pondérée par l'inverse d'un certain nombre de sommets parmi les point d'intersection avec des données non tronquées afin de générer la valeur de pondération.

7. Procédé selon la revendication 2 dans lequel les étapes de reconstruction comprennent la rétroprojection de chacune des sommets en une région volumétrique tridimensionnelle de la représentation d'image volumétrique de sorte que chaque somme rétroprojetée contribue à une pluralité de plans parallèles de la représentation d'image volumétrique.

8. Procédé selon la revendication 7, dans lequel l'étape de rétroprojection comprend :
- la détermination d'un segment volumétrique de la représentation d'image volumétrique affecté par chaque ensemble de données rétroprojetées bidimensionnelles;
- la génération d'adresses de voxels pour la représentation d'image volumétrique tridimensionnelle correspondant au volume affecté;
- la coordination d'un système de coordonnées de la représentation d'image volumétrique et de chaque ensemble de données bidimensionnelles;
- l'adressage de l'ensemble de données bidimensionnelles pour récupérer des valeurs de données correspondant à chaque adresse de voxel générée;
- la pondération de chaque valeur de données récupérée dans l'ensemble de données bidimensionnelles; et
- la récupération d'une valeur de données stockée dans la représentation d'image volumétrique correspondant à chaque adresse de voxel générée et l'addition de la valeur de données pondérée correspondante provenant de l'ensemble de données bidimensionnelles à celle-ci.

9. Appareil d'imagerie diagnostique qui comprend :
- une source de rayonnement (16) prévue pour générer un faisceau conique (20) de rayonnement,
- un moyen prévu pour déplacer la source de rayonnement et un sujet l'un par rapport à l'autre de sorte qu'un sommet du faisceau conique (20) de rayonnement se déplace le long d'une trajectoire spiralée;
- un détecteur de rayonnement (30) prévu pour convertir le rayonnement qui est passé à travers le sujet en une pluralité de vues bidimensionnelles de faisceaux coniques partiels, chaque vue de faisceau conique partiel étant non tronquée dans une direction parallèle à une tangente à la trajectoire spiralée au sommet et étant tronquée dans une autre direction, et
- des moyens de reconstruction (44, 46, 48, 50, 52, 54) prévus pour traiter les vues de faisceaux coniques partiels et rétroprojeter les vues de faisceaux coniques partiels traitées en une représentation d'image volumétrique, les moyens de reconstruction comprenant :
* un moyen à processeur (44) fourni pour calculer une vue pondérée pour chacune des vues de faisceaux coniques partiels;
* un moyen à processeur (48) prévu pour calculer une première composante bidimensionnelle (Q1) qui est proportionnelle à une partie constante d'une fonction de pondération redondante, essentiellement par convolution unidimensionnelle de la vue pondérée le long de la direction de la tangente à la trajectoire spiralée au sommet avec un filtre de reconstruction;
* un moyen à processeur (46, 50) prévu pour calculer une seconde composante bidimensionnelle (Q2), qui correspond à une partie non constante de la fonction de pondération redondante, essentiellement par rétroprojection bidimensionnelle sur la trace du point de reconstruction d'une dérivée partielle d'une dérivée partielle pondérée des intégrales linéaires de la vue pondérée, la vue pondérée étant multipliée par la partie non constante de la fonction de pondération redondante, la partie non constante de la fonction de pondération redondante étant calculée sur base des points d'intersection d'un plan avec une partie de la trajectoire spiralée, et
* des moyens à rétroprojecteur (52, 54) pour rétroprojeter la première composante et la seconde composante dans une partie affectée de la représentation d'image volumétrique.

10. Appareil selon la revendication 9 dans lequel les moyens de reconstruction sont aménagés pour reconstruire les vues de faisceaux coniques partiels sur la base de plans et d'emplacements de voxels définis de la représentation d'image volumétrique de sorte que les vues de faisceaux coniques partiels contribuent à des plans multiples pour générer la représentation d'image volumétrique.

11. Appareil selon l'une quelconque des revendications 9 ou 11 dans lequel le détecteur de rayonnement est aménagé :
- pour recevoir le rayonnement sur une matrice bidimensionnelle d'éléments détecteurs; et
- pour être échantillonné sur une pluralité d'incréments angulaires le long d'un segment d'arc spiralé de la trajectoire spiralée déplacée par le sommet pour générer une pluralité de matrices de valeurs de données bidimensionnelles, chacune des valeurs de données correspondant à un élément détecteur et chacune des matrices correspondant à l'une des vues de faisceaux coniques partiels;
et les moyens de reconstruction étant aménagés :
- pour pondérer les valeurs de données de chaque matrice bidimensionnelle selon le cosinus d'un angle compris entre un rayon ayant son origine au sommet et correspondant à l'élément détecteur et un rayon ayant son origine au sommet et étant normal à un plan de coordonnées locales au niveau du détecteur de rayonnement;
- pour multiplier chaque valeur des secondes composantes selon un carré de la distance entre le sommet et l'élément détecteur correspondant à la valeur de données;
- pour additionner la première composante et la seconde composante;
et les moyens rétroprojecteurs étant aménagés pour projeter en trois dimensions la somme dans une mémoire d'images volumétriques.

12. Appareil selon la revendication 9 dans lequel les moyens de reconstruction sont aménagés:
- pour calculer une dérivée partielle pondérée cosinusoïdale des intégrales linéaires.

13. Appareil selon la revendication 9 dans lequel les moyens de reconstruction comprennent des moyens à processeur (70, 72, 74, 82, 84, 86, 88) pour calculer une fonction de pondération, lesquels moyens à processeur étant aménagés :
- pour déterminer un sous-volume de la représentation d'image volumétrique affecté par chacune des vues de faisceaux coniques partiels; et
- pour calculer les traces de points terminaux de la plage de rétroprojection pour chaque tranche dans la représentation d'image volumétrique, et un moyen de multiplication par une valeur de pondération sur la base de la trace pour générer la fonction de pondération.

14. Appareil selon la revendication 13 dans lequel les moyens à processeur (70, 72, 74, 82, 84 , 86, 88) pour calculer la valeur de pondération sont encore aménagés :
- pour calculer des projections orthogonales des traces;
- pour calculer un certain nombre d'intersections entre le segment d'arc spiralé et un plan défini par la première dérivée des intégrales linéaires;
- pour déterminer le nombre de fois que le plan coupe le segment d'arc spiralé;
- pour déterminer des intersections des sommets avec des données non tronquées le long du plan; et
- pour régler la fonction de pondération sur zéro pour les données tronquées et pour régler la fonction de pondération égale à une valeur qui est proportionnelle à 1 moins le nombre d'intersections divisée par une constante et pondérée par l'inverse d'un certain nombre de sommets parmi les intersections avec des données non tronquées pour générer la valeur de pondération.

15. Appareil selon l'une quelconque des revendications 9 à 14 dans lequel le processeur de reconstruction est aménagé pour rétroprojeter chacune des vues de faisceaux coniques partiels dans une région volumétrique tridimensionnelle d'une représentation d'image volumétrique de sorte que chaque vue rétroprojetée contribue à une pluralité de plans parallèles de la représentation d'image volumétrique.

16. Appareil selon la revendication 15 dans lequel le processus de reconstruction est aménagé :
- pour déterminer un segment volumétrique de la représentation d'image volumétrique affectée par chaque ensemble de données rétroprojetées bidimensionnelles;
- pour générer des adresses de voxels pour la représentation d'image volumétrique bidimensionnelle correspondant au volume affecté;
- pour coordonner un système de coordonnées de la représentation d'image volumétrique et de chaque ensemble de données bidimensionnelles;
- pour adresser l'ensemble de données bidimensionnelles afin de récupérer des valeurs de données correspondant à chaque adresse de voxel générée;
